# EUROPEAN PATENT SPECIFICATION

(11) **EP 4 267 184 B1**
(45) Date of publication and mention of the grant of the patent: **26.11.2025**
(21) Application number: 21844228.3
(22) Date of filing: 20.12.2021
(51) Int. Cl.: A61K 39/395, C07K 16/24

(54) **INTERLEUKIN 5 BINDING PROTEIN DOSAGE REGIMEN**
INTERLEUKIN 5 BINDENDES PROTEIN-DOSIERUNGSREGELN
RÉGIME DE DOSAGE DE LA PROTÉINE DE LIAISON DE L'INTERLEUKINE 5

(30) Priority: 22.12.2020 US 202063128922 P; 25.05.2021 US 202163192854 P; 09.07.2021 US 202163220182 P; 03.12.2021 US 202163285513 P
(43) Date of publication of application: 01.11.2023
(60) Divisional application: 25195590.2
(73) Proprietor: GlaxoSmithKline Intellectual Property Development Limited, Stevenage SG1 2NY (GB)
(72) Inventor: AUSTIN, Daren J, Brentford Middlesex TW8 9GS (GB); BIRD, Nicholas P, Stevenage Hertfordshire SG1 2NY (GB); MONCK, Myrna A, Collegeville, Pennsylvania 19426 (US); POULIQUEN, Isabelle J, Brentford Middlesex TW8 9GS (GB); SHUMAN, Melissa A, Collegeville, Pennsylvania 19426 (US)
(74) Representative: Suen, Victoria Melissa
(86) International application number: PCT/EP2021/086689
(87) International publication number: WO 2022/136209

(56) References cited:
- WO-A1-2018/215964
- WO-A1-2018/215964
- WO-A1-2019/224724
- WO-A1-2019/224724

## Description

### FIELD OF THE INVENTION

The present invention relates to an antibody, or pharmaceutical compositions comprising said antibody, for use in the treatment of IL-5 mediated diseases by a novel dose or dosage regimen.

### BACKGROUND TO THE INVENTION

Asthma is a chronic heterogeneous lung disease characterised by inflammation, narrowing of the airways, and reversible airway obstruction. Patients with uncontrolled asthma suffer from continuing symptoms and acute exacerbations of the disease. Exacerbations are particularly disabling, necessitating patient treatment usually with oral/systemic corticosteroids. The frequency of asthma exacerbations appears to be closely related to eosinophil airway inflammation (FitzGerald & Gibson, Prevention. Thorax. 2006; 61: 992-999).

Persistent eosinophil inflammation is a feature of more than 50% of patients with severe asthma (Chung et al. Eur. Respir. J. 2014; 43: 343-73). Several monoclonal antibodies targeting eosinophil inflammation have received marketing authorization for asthma with an eosinophilic phenotype, including 3 targeting either interleukin-5 (IL-5) or its receptor (IL-5R): mepolizumab (Nucala), reslizumab (Cinqair) and benralizumab (Fasenra). All three, by utilizing blood eosinophils as a biomarker to predict patients likely to respond to therapy, have been shown to reduce asthma exacerbations, and improve lung function and health-related quality of life, in patients with asthma with an eosinophilic phenotype.

IL-5 is a secreted protein. IL-5 plays a role in a number of different diseases such as asthma, mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma, chronic obstructive pulmonary disease, eosinophilic granulomatosis with polyangiitis (EGPA) , hypereosinophilic syndrome (HES), nasal polyposis (NP), bullous pemphigoid, eosinophilic esophagitis, atopic dermatitis, moderate atopic dermatitis and severe atopic dermatitis and chronic rhinosinusitis with nasal polyps (CRSwNP), Inflammatory bowel disease (IBD), and allergic bronchopulmonary aspergillosis (ABPA) . These serious diseases affect hundreds of millions of people world wide.

In light of the above, it is clear that there is a need for more effective treatments for asthma. In particular, there exists a need for an efficacious treatment for IL-5 mediated disease such as eosinophilic asthma, that has a reduced dosing profile.

Hence the present disclosure provides such compositions in a novel dose, dosage regimens and methods of treatment suitable for treating IL-5 mediated disease such as asthma, mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma as well as Inflammatory bowel disease (IBD), allergic bronchopulmonary aspergillosis (ABPA) , eosinophilic granulomatosis with polyangiitis (EGPA) , hypereosinophilic syndrome (HES), nasal polyposis (NP), chronic rhinosinusitis with nasal polyps (CRSwNP) and dermatitis which are both efficacious and have a reduced dosing profile.

WO2018/215964 relates to antibodies for treating IL-5 mediated diseases, and discloses the heavy chain and light chain sequences of the antibody 28Y042-7F11-1.

### SUMMARY OF THE INVENTION

The present invention is directed to an antibody which binds to IL-5 comprising a heavy chain and a light chain, wherein the heavy chain comprises a heavy chain variable region sequence having the amino acid sequence shown in SEQ ID NO: 3; and the light chain comprises a light chain variable region sequence having the amino acid sequence shown in SEQ ID NO: 4, wherein the antibody comprises a heavy chain Fc domain having a tyrosine residue at position 252, a threonine residue at position 254 and a glutamic acid residue at position 256, wherein the heavy chain Fc domain is a human IgG1 Fc domain, for use in the treatment of an IL-5 mediated disease, wherein the antibody is to be administered subcutaneously to a human subject at a dose of about 100 mg to about 300 mg, at a frequency of about once every 6 months or once every 26 weeks (Q26W).

In a first aspect, the present disclosure provides a pharmaceutical composition comprising from about 100 mg to about 300 mg of an antigen binding protein which binds to IL-5, wherein the antigen binding protein comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, and a pharmaceutically acceptable excipient.

In a further aspect, the present disclosure provides an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, for use in the treatment of an IL-5 mediated disease, by administration to a subject at a dose of about 100 mg to about 300 mg, at a frequency of about once every 6 months.

In a further aspect, the present disclosure provides a pharmaceutical composition for use in the treatment of an IL-5 mediated disease wherein the composition comprises from about 100 mg to about 300 mg of an antigen binding protein which binds to IL-5, wherein the antigen binding protein comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, and wherein the pharmaceutical composition is for administration to a subject about once every 6 months.

In a further aspect, the present disclosure provides a method of treating an IL-5 mediated disease comprising administering to a subject in need thereof an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months.

In a further aspect, the present disclosure provides a method of treating an IL-5 mediated disease in a subject comprising the steps of:
a) identifying a subject with a disease selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma; and
b) administering about 100 mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, to the subject about once every 6 months;
whereby the disease in the subject is treated.

In a further aspect, the present disclosure provides a method of decreasing an absolute blood eosinophil count in a subject comprising the steps of:
a) identifying a subject having a condition selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma; and
b) administering to the subject an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months,
whereby the absolute blood eosinophil count in the subject is decreased.

In a further aspect, the present disclosure provides a pre-filled syringe comprising:
a) about 100mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region; and
b) a pharmaceutically acceptable excipient.

In some embodiments, disclosed herein are compositions comprising about 100 mg of an antigen binding protein disclosed herein. In some embodiments, the method comprises administering about 100 mg of the antigen binding protein.

In some embodiments, the heavy chain variable region of the antigen binding protein further comprises a heavy chain FR4 amino acid sequence as shown in SEQ ID NO: 19.

In some embodiments, the heavy chain Fc domain is an IgG1 Fc domain. In further embodiments, the heavy chain Fc domain is a human IgG1 Fc domain.

In some embodiments, the antigen binding protein comprises: a heavy chain variable region sequence having the amino acid sequence shown in SEQ ID NO: 3; and a light chain variable region sequence having the amino acid sequence shown in SEQ ID NO: 4.

In some embodiments, the antigen binding protein is an antibody comprising a heavy chain having the amino acid sequence shown in SEQ ID NO: 1 and a light chain having the amino acid sequence shown in SEQ ID NO: 2.

In some embodiments, the composition is for subcutaneous administration.

In some embodiments, the composition is administered once every 6 months. In some embodiments, the composition is administered once every 26 weeks (Q26W).

In some embodiments, the composition is administered to a human subject.

In some embodiments, the subject (prior to treatment) has an absolute blood eosinophil count of greater than or equal to 150 cells per µL. In further embodiments, the subject (prior to treatment) has an absolute blood eosinophil count of greater than or equal to 200 cells per µL. In other embodiments the subject (prior to treatment) has a blood eosinophil count which is greater than or equal to 300 eosinophils per uL of blood in the past 12 months.
In further embodiments the subject may also have high-sensitivity C-reactive protein (hsCRP) <10 mg L⁻¹.

In some embodiments, the composition comprises an aqueous liquid formulation at about pH 6.0 containing the antigen binding protein and histidine, trehalose, arginine, EDTA and/or polysorbate 80. In some embodiments, the composition comprises an aqueous liquid formulation at about pH 6.0 containing the antigen binding protein and histidine, trehalose, arginine, EDTA and polysorbate 80. In a further embodiment, the composition comprises an aqueous liquid formulation at about pH 6.0 containing the antigen binding protein and about 20 mM histidine, about 180 mM trehalose, about 40 mM arginine, about 0.05 mM EDTA and about 0.02% weight of polysorbate 80 to volume.

In some embodiments, the IL-5 mediated disease is asthma. In further embodiments, the asthma is selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma. Preferably, the asthma is asthma with an eosinophilic phenotype (i.e., mild eosinophilic asthma, moderate eosinophilic asthma or severe eosinophilic asthma, in particular severe eosinophilic asthma). In a further embodiment, the asthma is severe asthma.

In some embodiments, the IL-5 mediated disease is selected from: Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) and dermatitis.

### DESCRIPTION OF DRAWINGS/FIGURES

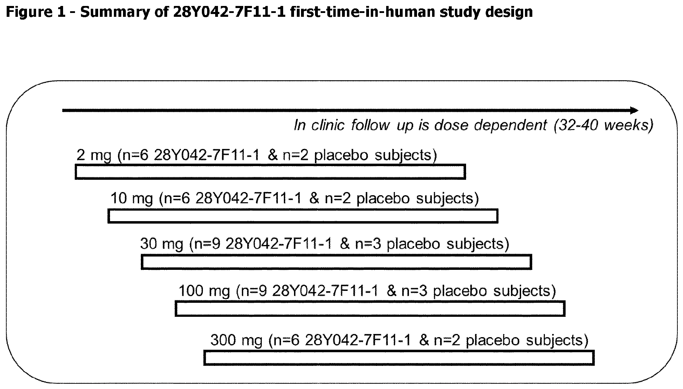

**FIGURE 1****: Graphical summary of study design.** Planned single ascending subcutaneous (SC) doses of 28Y042-7F11-1 were 2, 10, 30, 100 and 300 mg. Actual dose levels beyond the 2 mg starting dose were confirmed based on the emerging data. Planned cohort sizes are shown; additional participants were to be added to cohorts, if necessary, to characterise the pharmacokinetics or pharmacodynamics of 28Y042-7F11-1. In each cohort, participants were randomised 3:1 to receive 28Y042-7F11-1 or placebo. Additional cohorts of up to 12 participants were to be added to test additional dose levels or to repeat a dose level already tested, if deemed necessary; however, the dose was not to exceed 300 mg. The planned sample size was 48 participants; the maximum sample size allowed was n=72 participants (excluding replacements for prematurely withdrawn participants).

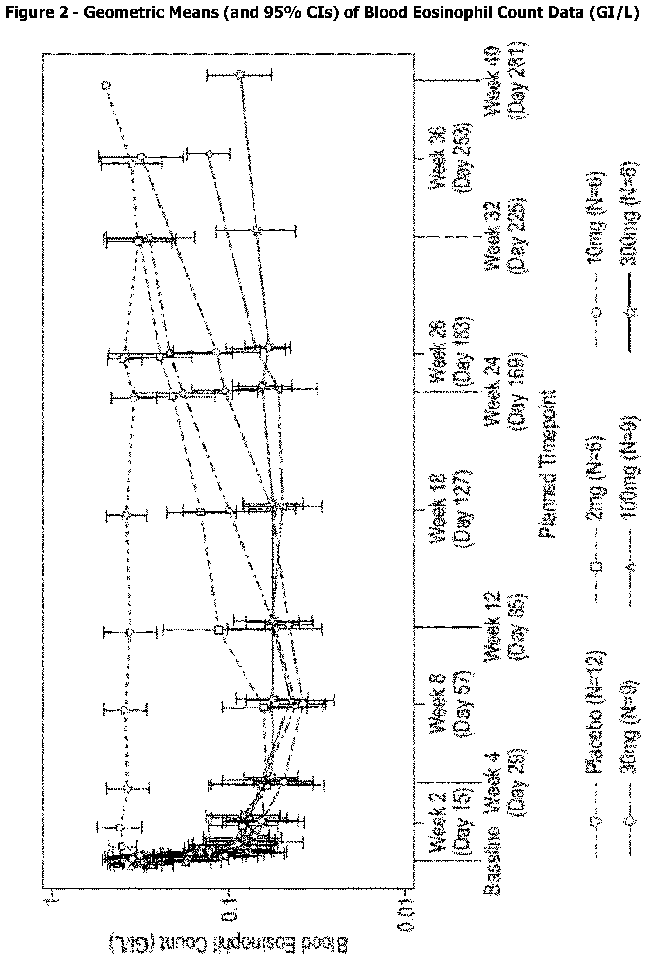

**FIGURE 2****: Geometric Means (and 95% CIs) of Blood Eosinophil Count Data (GI/L).** Note: 95% Confidence Interval (CI) is displayed only when at least 3 participants contribute to the statistical analysis.

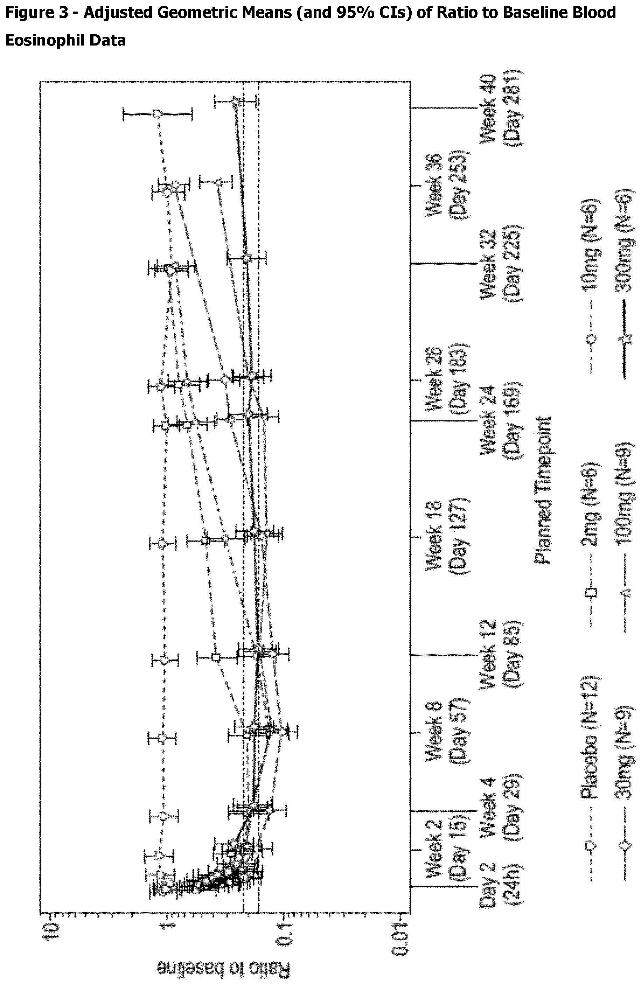

**FIGURE 3****: Adjusted Geometric Means (and 95% CIs) of Ratio to Baseline Blood Eosinophil Data.** Analysis was performed using an MMRM model on log-transformed data, with fixed categorical effects of treatment, planned time point and treatment-by-planned time point interaction and fixed continuous covariates of log Baseline blood eosinophil count and log Baseline blood eosinophil count-by-planned time point interaction. A Toeplitz covariance structure was used. Note: Reference line at ratio=0.22 indicates a 78% reduction from Baseline, as observed in the Phase 3b mepolizumab MUSCA trial. Reference line at ratio=0.16 indicates an 84% reduction from Baseline, as observed in the mepolizumab MENSA pivotal Phase 3 trial.

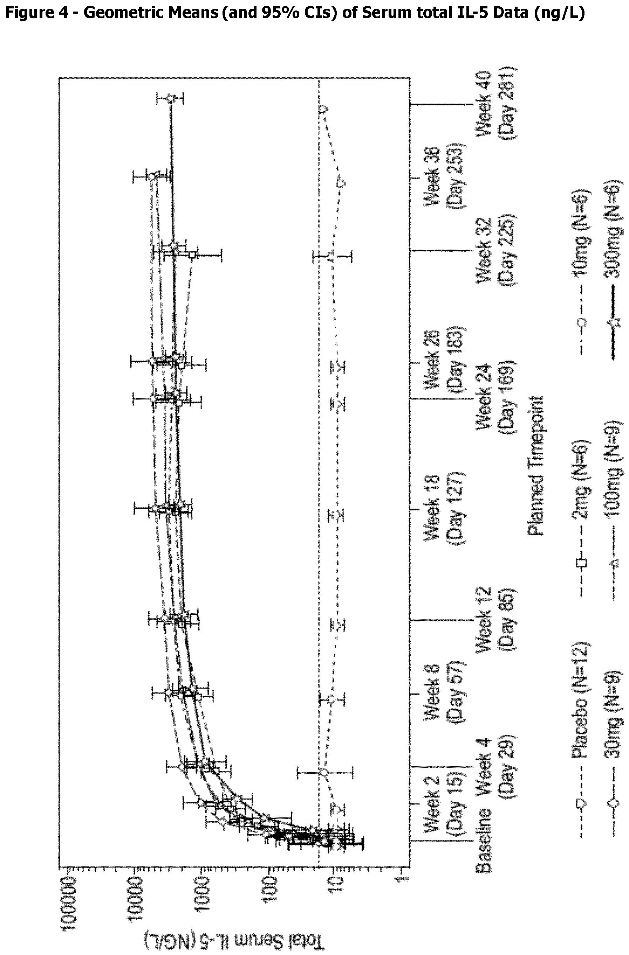

**FIGURE 4****: Geometric Means (and 95% CIs) of Serum total IL-5 Data (ng/L).** Values below the lower limit of quantification (LLQ=16.38 ng/L) were imputed to LLQ/2. LLQ shown by horizontal reference line. Note: 95% CI is only displayed when at least 3 participants contribute to the statistics and at least one value is not below the lower limit of quantification.

### DETAILED DESCRIPTION OF THE INVENTION

### DEFINITIONS

Unless defined otherwise, all technical and scientific terms used herein have the same meaning as commonly understood by one of ordinary skill in the art.

The term "antigen binding polypeptide" as used herein refers to antibodies, antibody fragments and other protein constructs which are capable of binding to an antigen.

The term "antibody" is used herein in the broadest sense to refer to molecules with an immunoglobulin-like domain (for example IgG, IgM, IgA, IgD or IgE) and includes monoclonal, recombinant, polyclonal, chimeric, human, humanised, multispecific antibodies, including bispecific antibodies, and heteroconjugate antibodies; a single variable domain (*e.g.* a domain antibody (DAB)), antigen binding antibody fragments, Fab, F(ab')₂, Fv, disulphide linked Fv, single chain Fv, disulphide-linked scFv, diabodies, TANDABS, etc. and modified versions of any of the foregoing (for a summary of alternative "antibody" formats see Holliger and Hudson, Nature Biotechnology, 2005, Vol 23, No. 9, 1126-1136). Alternative antibody formats are also contemplated and include alternative scaffolds in which the one or more CDRs of the antigen binding protein can be arranged onto a suitable non-immunoglobulin protein scaffold or skeleton, such as an affibody, a SpA scaffold, an LDL receptor class A domain, an avimer or an EGF domain.

The antibody may be derived from rat, mouse, primate (e.g., cynomolgus, Old World monkey or Great Ape), human or other sources such as nucleic acids generated using molecular biology techniques which encode an antibody molecule.

The antibody may comprise a constant region, which may be of any isotype or subclass. The constant region may be of the IgG isotype, for example, IgG₁, IgG₂, IgG₃, IgG₄ or variants thereof. The antigen binding protein constant region may be IgG₁.

The antigen binding protein may comprise one or more modifications selected from a mutated constant domain such that the antibody has enhanced effector functions/ADCC and/or complement activation.

The term "antibody variant" as used herein means an antibody that differs from a parent antibody by virtue of at least one amino acid modification (e.g., by having a different amino acid side chain), post-translational modification or other modification in at least one heavy chain, light chain, or combinations of these that results in a structural change (e.g., different amino acid side chain, different post-translational modification or other modification) relative to the parent antibody. Structural changes can be determined directly by a variety of methods well known in the art such as LC-MS, direct sequencing or indirectly via methods such as isoelectric focusing and the like. Such methods are well known to those of ordinary skill in the art.

In particular, references herein to "antigen binding proteins of the disclosure" refer to an antigen binding protein which binds to IL-5 and comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region and also any of the variants or specific embodiments of such antigen binding proteins as are described herein. The numbering of the amino acids in the heavy chain Fc domain (i.e. a tyrosine residue at position 252, a threonine residue at position 254 and a glutamic acid residue at position 256) was derived using EU numbering, as described in: Edelman et al. (1969) Proc. Natl. Acad. USA, 63: 78-85 [PMID: 5257969].

The antigen binding proteins of the disclosure can be used in any of the pharmaceutical compositions, dosage regimens, or method of treatments of the disclosure. The antigen binding proteins of the disclosure can be antibodies, for example IgG1 antibodies.

The term "about" or "approximately" can mean within an acceptable error range for the particular value as determined by one of ordinary skill in the art, which will depend in part on how the value is measured or determined, e.g ., the limitations of the measurement system.

For example, "about" can mean plus or minus 10%, per the practice in the art. Alternatively, "about" can mean a range of plus or minus 20%, plus or minus 10%, plus or minus 5%, or plus or minus 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value should be assumed. Also, where ranges and/or subranges of values are provided, the ranges and/or subranges can include the endpoints of the ranges and/or subranges. The term "interleukin-5" or "IL-5" as used herein includes human IL-5 comprising the amino acid sequence shown in SEQ ID NO: 11. The term "interleukin-5 receptor" or "IL-5R" as used herein includes human IL-5 Receptor Subunit Alpha Isoform 1 comprising the amino acid sequence shown in SEQ ID NO: 12.

The term "specifically binds", as used herein in relation to antigen binding proteins means that the antigen binding protein binds to a target antigen as well as a discrete domain, or discrete amino acid sequence, within a target antigen with no or insignificant binding to other (for example, unrelated) proteins. This term, however, does not exclude the fact that the antigen binding proteins may also be cross-reactive with closely related molecules (for example, those with a high degree of sequence identity or from another genera or species). The antigen binding proteins described herein may bind to human IL-5 with at least 2, 5, 10, 50, 100, or 1000-fold greater affinity than they bind to closely related molecules.

The binding affinity (K_{D}) of the antigen binding protein-target antigen interaction may be 1 mM or less, 100 nM or less, 10 nM or less, 2 nM or less or 1 nM or less. Alternatively, the K_{D} may be between 5 and 10 nM; or between 1 and 2 nM. The K_{D} may be between 1 pM and 500 pM; or between 500 pM and 1 nM. The binding affinity of the antigen binding protein is determined by the association constant (Kₐ) and the dissociation constant (K_{d}) (K_{D} = K_{d}/Kₐ). The binding affinity may be measured by BIACORE^{™}, for example, by capture of the test antibody onto a protein-A coated sensor surface and flowing target antigen over this surface. Alternatively, the binding affinity can be measured by FORTEBIO, for example, with the test antibody receptor captured onto a protein-A coated needle and flowing target antigen over this surface.

The K_{d} may be 1×10⁻³ Ms⁻¹ or less, 1×10⁻⁴ Ms⁻¹ or less, or 1×10⁻⁵ Ms⁻¹ or less. The K_{d} may be between 1×10⁻⁵ Ms⁻¹ and 1×10⁻⁴ Ms⁻¹; or between 1×10⁻⁴ Ms⁻¹ and 1×10⁻³ Ms⁻¹. A slow K_{d} may result in a slow dissociation of the antigen binding protein-target antigen complex and improved neutralization of the target antigen.

The term "specific antigen binding activity" as used herein means antigen binding activity as measured by Surface Plasmon Resonance (SPR). IL-5 specific binding activity may be determined by SPR using a BIACORE^{™} instrument, for example performed in the binding mode. It is binding activity divided by total protein (e.g., 28Y042-7F11-1) content in a sample.

The term "FcRn binding activity" as used herein means Neonatal Fc (FcRn) Receptor binding activity as measured by Surface Plasmon Resonance (SPR). FcRn binding may be determined using a BIACORE^{™} instrument. It is binding activity to the FcRn receptor, divided by the total protein concentration of the sample.

The SPR method for specific antigen binding and FcRn binding uses a reference standard of 28Y042-7F11-1. The 28Y042-7F11-1 reference standard can be used in assays to obtain system suitability and sample comparability data, to ensure methods are performing appropriately. The reference standard can allow the establishment of a calibration curve and concentrations of the samples are interpolated from the curve.

By "isolated", it is intended that the molecule, such as an antigen binding protein, is removed from the environment in which it may be found in nature. For example, the molecule may be purified away from substances with which it would normally exist in nature. For example, the mass of the molecule in a sample may be 95% of the total mass.

The terms "VH" and "VL" are used herein to refer to the heavy chain variable region and light chain variable region, respectively, of an antigen binding protein.

"CDRs" are defined as the complementarity determining region amino acid sequences of an antigen binding protein. These are the hypervariable regions of immunoglobulin heavy and light chains. There are three heavy chain and three light chain CDRs (or CDR regions) in the variable portion of an immunoglobulin. Thus, "CDRs" as used herein refers to all three heavy chain CDRs, all three light chain CDRs, all heavy and light chain CDRs, or at least one CDR and wherein the at least one CDR is CDRH3. Framework regions follow each of these CDR regions. Acceptable heavy chain variable region and light chain variable region framework 1, framework 2 and framework 3 regions are readily recognized by those of ordinary skill in the art. Acceptable heavy chain constant regions (including hinge regions) and light chain constant regions are readily recognized by those of ordinary skill in the art as well. Acceptable antibody isotypes are similarly readily recognized by those of ordinary skill in the art.

Throughout this specification, amino acid residues in variable domain sequences and full length antibody sequences are numbered according to the Kabat numbering convention. Similarly, the terms "CDR", "CDRL1", "CDRL2", "CDRL3", "CDRH1", "CDRH2", "CDRH3" used in the specification follow the Kabat numbering convention.

It will be apparent to those skilled in the art that there are alternative numbering conventions for amino acid residues in variable domain sequences and full length antibody sequences. There are also alternative numbering conventions for CDR sequences, for example those set out according to the Chothia numbering convention. The structure and protein folding of the antibody may mean that other residues are considered part of the CDR sequence and would be understood to be so by a skilled person.

Other numbering conventions for CDR sequences available to a skilled person include "AbM" (University of Bath) and "contact" (University College London) methods. The minimum overlapping region using at least two of the Kabat, Chothia, AbM and contact methods can be determined to provide the "minimum binding unit". The minimum binding unit may be a sub-portion of a CDR.

The term "asthma" as used herein means an inflammatory disease of the airways characterized by reversible airflow obstruction and bronchospasm. Common symptoms include wheezing, coughing, chest tightness, and shortness of breath. Asthma is a heterogeneous disease, usually characterized by chronic airway inflammation. It is defined by the history of respiratory symptoms such as wheeze, shortness of breath, chest tightness and cough that vary over time and in intensity, together with variable expiratory airflow limitation.

In the methods of the disclosure, a diagnosis of asthma in a subject may be made according to the guidance provided by the Global Initiative for Asthma (GINA) the Global Strategy for Asthma Management and Prevention (2016 update) document. Those of ordinary skill in the art will be familiar with the GINA diagnostic flow chart for clinical practice and diagnostic criteria for asthma in adults, adolescents and children 6-11 years (Table 1) shown below as well as other aspect of the guidance (*e.g.,* for pregnant women *etc.*)*.* See also Table 2 and Table 3.

**Table 1.**

| Box 1-2. Diagnostic criteria for asthma in adults, adolescents, and children 6-11 years. | |
|---|---|
| Asthma is a heterogeneous disease, usually characterized by chronic airway inflammation. It is defined by the history of respiratory symptoms such as wheeze, shortness of breath, chest tightness and cough that vary over time and in intensity, together with variable expiratory airflow limitation. | |
| DIAGNOSTIC FEATURE | DIAGNOSTIC FEATURE |
| 1. History of variable respiratory symptoms | |
| Wheeze, shortness of breath, chest tightness and cough | • Generally more than one type of respiratory symptom (in adults, isolated cough is seldom due to asthma) |
| Descriptors may vary between cultures and by age, e.g. children may be described as having heavy breathing | |
| | • Symptoms occur variably over time and vary in intensity |
| | • Symptoms are often worse at night or on waking |
| | • Symptoms are often triggered by exercise, laughter, allergens, cold air |
| | • Symptoms often appear or worsen with viral infections |

| 2. Confirmed variable expiratory airflow limitation | |
|---|---|
| Documented excessive variability in lung function* (one or more of the tests below) AND documented airflow limitation* | The greater the variations, or the more occasions excess variation is seen, the more confident the diagnosis |
| | At least once during diagnostic process when FEV1 is low, confirm that FEV1/FVC is reduced (normally >0.75-0.80 in adults, >0.90 in children) |
| Positive bronchodilator (BD) reversibility test* (more likely to be positive if BD medication is withheld before test: SABA ≥4 hours, LABA ≥15 hours) | Adults: increase in FEV1 of >12% and >200 mL from baseline, 10-15 minutes after 200-400 mcg albuterol or equivalent (greater confidence if increase is >15% and >400 mL). |
| | Children: increase in FEV1 of >12% predicted |
| Excessive variability in twice-daily PEF over 2 weeks* | Adults: average daily diurnal PEF variability > 10%** |
| | Children: average daily diurnal PEF variability > 13%** |
| Significant increase in lung function after 4 weeks of anti-inflammatory treatment | Adults: increase in FEV1 by >12% and >200 mL (or PEF† by >20%) from baseline after 4 weeks of treatment, outside respiratory infections |
| Positive exercise challenge test* | Adults: fall in FEV1 of >10% and >200 mL from baseline |
| | Children: fall in FEV1 of >12% predicted, or PEF >15% |
| Positive bronchial challenge test (usually only performed in adults) | Fall in FEV1 from baseline of ≥20% with standard doses of methacholine or histamine, or ≥15% with standardized hyperventilation, hypertonic saline or mannitol challenge |
| Excessive variation in lung function between visits* (less reliable) | Adults: variation in FEV1 of >12% and >200 mL between visits, outside of respiratory infections |
| | Children: variation in FEV1 of >12% in FEV1 or >15% in PEF† between visits (may include respiratory infections) |
| BD: bronchodilator (short-acting SABA or rapid-acting LABA); FEV1: forced expiratory volume in 1 second; LABA: long-acting beta2-agonist; PEF: peak expiratory flow (highest of three readings); SABA: short-acting beta2-agonist. See Box 1-4 for diagnosis in patients already taking controller treatment. | |
| *These tests can be repeated during symptoms or in the early morning. **Daily diurnal PEF variability is calculated from twice daily PEF as ([day's highest minus day's lowest] / mean of day's highest and lowest), and averaged over one week. †For PEF, use the same meter each time, as PEF may vary by up to 20% between different meters. BD reversibility may be lost during severe exacerbations or viral infections. If bronchodilator reversibility is not present at initial presentation, the next step depends on the availability of other tests and the urgency of the need for treatment. In a situation of clinical urgency, asthma treatment may be commenced and diagnostic testing arranged within the next few weeks (Box 1-4), but other conditions that can mimic asthma (Box 1-3) should be considered, and the diagnosis of asthma confirmed as soon as possible. | |

**Table 2.**

| Box 1-3. Differential diagnosis of asthma in adults, adolescents and children 6-11 years. | | |
|---|---|---|
| Age | Condition | Symptoms |
| 6-11 years | Chronic upper airway cough syndrome | Sneezing, itching, blocked nose, throat-clearing |
| | Inhaled foreign body | Sudden onset of symptoms, unilateral wheeze |
| | Bronchiectasis | Recurrent infections, productive cough |
| | Primary ciliary dyskinesia | Recurrent infections, productive cough, sinusitis |
| | Congenital heart disease | Cardiac murmurs |
| | Bronchopulmonary dysplasia | Pre-term delivery, symptoms since birth |
| | Cystic fibrosis | Excessive cough and mucus production, gastrointestinal symptoms |
| 12-39 years | Chronic upper airway cough syndrome | Sneezing, itching, blocked nose, throat-clearing |
| | Vocal cord dysfunction | Dyspnea, inspiratory wheezing (stridor) |
| | | Dizziness, paresthesia, sighing |
| | Hyperventilation, dysfunctional breathing | |
| | Bronchiectasis | Productive cough, recurrent infections |
| | | Excessive cough and mucus production |
| | Cystic fibrosis | Cardiac murmurs Shortness of breath, family history of early emphysema |
| | Congenital heart disease | Sudden onset of symptoms |
| | Alpha1-antitrypsin deficiency | |
| | Inhaled foreign body | |
| 40+ years | Vocal cord dysfunction | Dyspnea, inspiratory wheezing (stridor) |
| | Hyperventilation, dysfunctional breathing | Dizziness, paresthesia, sighing |
| | COPD* | Cough, sputum, dyspnea on exertion, smoking or noxious exposure |
| | | Productive cough, recurrent infections |
| | Bronchiectasis | Dyspnea with exertion, nocturnal symptoms |
| | Cardiac failure | Treatment with angiotensin converting enzyme (ACE) inhibitor |
| | Medication-related cough | Dyspnea with exertion, non-productive cough, finger clubbing |
| | Parenchymal lung disease | Sudden onset of dyspnea, chest pain |
| | | Dyspnea, unresponsive to bronchodilators |
| | Pulmonary embolism | |
| | Central airway obstruction | |
| *Any of the above conditions may also contribute to respiratory symptoms in patients with confirmed asthma. | | |

**Table 3.**

| Box 1-4. Confirming the diagnosis of asthma in a patient already taking controller treatment. | |
|---|---|
| **Current status** | **Steps to confirm the diagnosis of asthma** |
| Variable respiratory symptoms and variable airflow limitation | Diagnosis of asthma is confirmed. Assess the level of asthma control and review controller treatment. |
| Variable respiratory symptoms but no variable airflow limitation | Repeat BD reversibility test again after withholding BD (SABA: 4 hours; LABA: 12+ hours) or during symptoms. If normal, consider alternative diagnoses (Box 1-3). |
| | *If FEV1 is >70% predicted:* consider a bronchial provocation test. If negative, consider stepping down controller treatment and reassess in 2-4 weeks |
| | *If FEV1 is <70% predicted:* consider stepping up controller treatment for 3 months, then reassess symptoms and lung function. If no response, resume previous treatment and refer patient for diagnosis and investigation |
| Few respiratory symptoms, normal lung function, and no variable airflow limitation | Repeat BD reversibility test again after withholding BD (SABA: 4 hours; LABA: 12+ hours) or during symptoms. If normal, consider alternative diagnoses (Box 1-3). |
| | Consider stepping down controller treatment: |
| | • *If symptoms emerge and lung function falls*: asthma is confirmed. Step up controller treatment to lowest previous effective dose. |
| | • *If no change in symptoms or lung function at lowest controller step*: consider ceasing controller, and monitor patient closely for at least 12 months. |
| Persistent shortness of breath and fixed airflow limitation | Consider stepping up controller treatment for 3 months, then reassess symptoms and lung function. If no response, resume previous treatment and refer patient for diagnosis and investigation. Consider asthma-COPD overlap syndrome. |
| BD: bronchodilator; LABA: long-acting beta2-agonist; SABA: short-acting beta2-agonist | |

In the methods of the disclosure "asthma" may be "mild asthma," "moderate asthma" or "severe asthma." In the methods of the disclosure asthma severity can be assessed according to the GINA guidance. In particular, asthma severity can be assessed retrospectively from the level of treatment required to control symptoms and exacerbations. For example, it can be assessed once the patient has been on controller treatment for several months and, if appropriate, treatment step down has been attempted to find the patient's minimum effective level of treatment. Asthma severity is not a static feature and may change over months or years.

Asthma severity can be assessed when the patient has been on regular controller treatment for several months:
- "Mild asthma" is asthma that is well controlled with Step 1 or Step 2 treatment (*see* Figure 9 of WO2018215964), *i.e.,* with as-needed reliever medication alone, or with low-intensity controller treatment such as low dose ICS, leukotriene receptor antagonists or chromones.
- "Moderate asthma" is asthma that is well controlled with Step 3 treatment (*see* Figure 9 of WO2018215964), *e.g.,* low dose ICS/LABA.
- "Severe asthma" is asthma that requires Step 4 or 5 treatment (*see* Figure 9 of WO2018215964), *e.g.,* high-dose ICS/LABA, to prevent it from becoming 'uncontrolled', or asthma that remains 'uncontrolled' despite this treatment. While many patients with uncontrolled asthma may be difficult to treat due to inadequate or inappropriate treatment, or persistent problems with adherence or comorbidities such as chronic rhinosinusitis or obesity, the European Respiratory Society/American Thoracic Society Task Force on Severe Asthma considered that the definition of "severe asthma" should be reserved for patients with refractory asthma and those in whom response to treatment of comorbidities is incomplete. Table 4 can also be referred to during the assessment of asthma severity.

**Table 4.**

| Box 3-6. Low, medium and high daily doses of inhaled corticosteroids. | | | |
|---|---|---|---|
| Drug | Daily dose (mcg) | | |
| | Low | Medium | High |
| Beclometasone dipropionate (CFC)* | 200-500 | >500-1000 | >1000 |
| Beclometasone dipropionate (HFA) | 100-200 | >200-400 | >400 |
| Budesonide (DPI) | 200-400 | >400-800 | >800 |
| Ciclesonide (HFA) | 80-160 | >160-320 | >320 |
| Fluticasone furoate (DPI) | 100 | n.a. | 200 |
| Fluticasone propionate(DPI) | 100-250 | >250-500 | >500 |
| Fluticasone propionate (HFA) | 100-250 | >250-500 | >500 |
| Mometasone furoate | 110-220 | >220-440 | >440 |
| Triamcinolone acetonide | 400-1000 | >1000-2000 | >2000 |

| Children 6-11 years (for children 5 years and younger) | | | |
|---|---|---|---|
| Beclometasone dipropionate (CFC)* | 100-200 | >200-400 | >400 |
| Beclometasone dipropionate (HFA) | 50-100 | >100-200 | >200 |
| Budesonide (DPI) | 100-200 | >200-400 | >400 |
| Budesonide (nebules) | 250-500 | >500-1000 | >1000 |
| Ciclesonide | 80 | >80-160 | >160 |
| Fluticasone furoate (DPI) | n.a. | n.a. | n.a. |
| Fluticasone propionate (DPI) | 100-200 | >200-400 | >400 |
| Fluticasone propionate (HFA) | 100-200 | >200-500 | >500 |
| Mometasone furoate | 110 | ≥220-<440 | ≥440 |
| Triamcinolone acetonide | 400-800 | >800-1200 | >1200 |
| CFC: chlorofluorocarbon propellant; DPI: dry powder inhaler; HFA: hydrofluoroalkane propellant; n.a. not applicable *Beclometasone dipropionate CFC is included for comparison with older literature | | | |

In the methods of the disclosure "asthma" may be "mild eosinophilic asthma," "moderate eosinophilic asthma," or "severe eosinophilic asthma."

"Mild eosinophilic asthma" is mild asthma with an eosinophilic phenotype. For example, subjects with mild eosinophilic asthma may have mild asthma and blood eosinophils greater than or equal to 150 eosinophils per µL of blood in the past 12 months, greater than or equal to 200 eosinophils per µL of blood in the past 12 months, greater than or equal to 300 eosinophils per µL of blood in the past 12 months or greater than or equal to 350 eosinophils per µL of blood in the past 12 months.

"Moderate eosinophilic asthma" is moderate asthma with an eosinophilic phenotype. For example, subjects with moderate eosinophilic asthma may have moderate asthma and blood eosinophils greater than or equal to 150 eosinophils per µL of blood in the past 12 months, greater than or equal to 200 eosinophils per µL of blood in the past 12 months, greater than or equal to 300 eosinophils per µL of blood in the past 12 months or greater than or equal to 350 eosinophils per µL of blood in the past 12 months.

"Severe eosinophilic asthma" is severe asthma with an eosinophilic phenotype. For example, subjects with severe eosinophilic asthma may have severe asthma and blood eosinophils greater than or equal to 150 eosinophils per µL of blood in the past 12 months, greater than or equal to 200 eosinophils per µL of blood in the past 12 months, greater than or equal to 300 eosinophils per µL of blood in the past 12 months (preferred) or greater than or equal to 350 eosinophils per µL of blood in the past 12 months.

Subjects with severe eosinophilic asthma may also meet, one or more of, the criteria described in Table 5.

**Table 5.**

| A subject has severe eosinophilic asthma if they meet the following criteria: |
|---|
| 1) The subject has clinical features of severe refractory asthma similar to those indicated in the American Thoracic Society Workshop on Refractory Asthma (162, Am. J. Respir. Crit. Care Med. 2341 (2000)) for ≥12 months. |
| 2) The subject has a well-documented requirement for regular treatment with high dose ICS (inhaled corticosteroids) (*i.e.,* ≥880 µg/day fluticasone propionate or equivalent daily), with or without maintenance OCS (oral corticosteroids), in the past 12 months. |
| 3) The subject has a well-documented requirement for controller medication, e.g., long-acting beta-2-agonist, leukotriene receptor antagonist or theophylline in the past 12 months. |
| 4) The subject has persistent airflow obstruction as indicated by a pre-bronchodilator FEV₁ <80% predicted recorded or peak flow diurnal variability of >20% on 3 or more days. |
| 5) The subject has airway inflammation which is likely to be eosinophilic in nature as indicated by one of the following characteristics at present or documented in the previous 12 months: - An elevated peripheral blood eosinophil level of ≥300/µL that is related to asthma or |
| - Sputum eosinophils ≥3% or |
| - Exhaled nitric oxide ≥50 ppb or |
| - Prompt deterioration of asthma control (based on documented clinical history or objective measures) following a ≤25% reduction in regular maintenance dose of inhaled or oral corticosteroid dose in the previous 12 months |
| 8) The subject has a previously confirmed history of two or more asthma exacerbations requiring treatment with oral or systemic corticosteroids in the prior 12 months prior, despite the use of high-dose ICS and additional controller medication. For subjects receiving maintenance OCS with high-dose ICS plus controller, the OCS treatment for exacerbations had to be a two-fold or greater increase in the dose of OCS. |
| 9) The subject has asthma as documented by either: |
| - Airway reversibility (FEV₁ ≥12% and 200 mL) at present or documented in the previous 12 months or |
| - Airway hyper-responsiveness (provocative concentration causing a 20% fall in FEV₁ of methacholine <8 mg/mL or provocative dose causing a 20% fall in FEV₁ of histamine <7.8 µmol) documented in the prior 12 months or |
| - Airflow variability in clinic FEV₁ ≥20% between two examinations documented in the prior 12 months (FEV₁ recorded during an exacerbation is not valid) or |
| - Airflow variability as indicated by >20% diurnal variability in peak flow observed on 3 or more days. |

Importantly, subjects with severe eosinophilic asthma according to these criteria may have less than 150 eosinophils per µL of blood at the initiation of treatment.

The term "pharmaceutical composition" as used herein means a composition suitable for administration to a patient.

The term "therapeutically effective amount" as used herein means an amount of an agent (such as an antigen binding protein or a pharmaceutical composition), which provides a therapeutic benefit in the treatment or management of one or more symptoms of a condition to be treated (such as asthma, mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma). Examples of such treatment or management of one or more symptoms of asthma-including asthma, mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma-include 1) a reduction of the frequency of asthma exacerbations; 2) a reduction in the time to first clinically significant exacerbation requiring oral or systemic corticosteroids, hospitalisation, and/or emergency department (ED) visits; 3) a reduction in the frequency of exacerbations requiring hospitalization (including intubation and admittance to an intensive care unit) or ED visits; 4) a reduction in the time to first exacerbation requiring hospitalization or ED visit; 5) a change from baseline in clinic pre-bronchodilator FEV1; 6) a change from baseline in clinic post-bronchodilator FEV1; 7) a change from baseline in an Asthma Control Questionnaire (ACQ) score; 8) improved lung function as assessed by spirometry (e.g., vital capacity (VC), forced vital capacity (FVC), forced expiratory volume (FEV) at timed intervals of 0.5, 1.0 (FEV1), 2.0, and 3.0 seconds, forced expiratory flow 25-75% (FEF 25-75) and maximal voluntary ventilation (MW) total lung capacity, idal volume, residual volume, expiratory reserve volume, inspiratory reserve volume, inspiratory capacity, inspiratory vital capacity, vital capacity, functional residual capacity, residual volume expressed as percent of total lung capacity, alveolar gas volume, actual volume of the lung including the volume of the conducting airway, forced vital capacity, etc.); and 9) a reduction in asthma exacerbations requiring steroids for control (such as oral steroids or steroids, such as prednisone, prednisolone etc., administered by any route). Such a reduction in asthma exacerbations requiring steroids for control may be an approximately 50% reduction in exacerbations requiring steroids (e.g., oral steroids).

Therapeutically effective amounts and treatment regimens are generally determined empirically and may be dependent on factors, such as the age, weight, and health status of the patient and disease or disorder to be treated. Such factors are within the purview of the attending physician.

As used herein, the term "treatment" refers to ameliorating or stabilising the specified condition, reducing or eliminating the symptoms of the condition, slowing or eliminating the progression of the condition, and preventing or delaying reoccurrence of the condition in a previously afflicted patient or subject.

The methods, antigen binding proteins and compositions of the disclosure need not affect a complete cure, or eradicate every symptom or manifestation of the disease to constitute a viable therapeutic treatment. As is recognised in the art, drugs employed as therapeutic agents in methods of treatment may reduce the severity of a given disease state, but need not abolish every manifestation of the disease to be regarded as useful therapeutic agents. Simply reducing the impact of a disease (for example, by reducing the number or severity of its symptoms, or by increasing the effectiveness of another treatment, or by producing another beneficial effect), or reducing the likelihood that the disease will occur (for example by delaying the onset of the disease) or worsen in a subject, is sufficient.

As used herein, the term "subject" refers to a human or animal body. The terms "individual", "subject" and "patient" are used herein interchangeably. The subject is typically a human. The subject may also be a mammal, such as a mouse, rat, or primate (e.g., a marmoset or monkey). The subject can be a non-human animal. The antigen binding proteins, compositions and methods of the disclosure also have veterinary use. The subject to be treated may be a farm animal, for example, a cow or bull, sheep, pig, ox, goat or horse, or may be a domestic animal such as a dog or cat. The animal may be any age, or a mature adult animal. The subject may be an adult or adolescent (≥12 years) subject.

In some embodiments, a subject described herein is a subject who has been switched from a treatment comprising an agent targeting IL-5 or IL-5R to a treatment comprising an antigen binding protein described herein. In some embodiments, a subject described herein is a subject who have been switched from a treatment comprising mepolizumab, reslizumab, or benralizumab to a treatment comprising an antigen binding protein described herein. In some embodiments, a subject described herein is a subject being treated with a nonbiologic treatment comprising an agent targeting IL-5 or IL-5R. In some embodiments, a subject described herein is a subject being treated with a nonbiologic treatment for asthma, for example corticosteroids and/or other controllers, such as long-acting beta-2-agonist (LABA) and/or long-acting muscarinic antagonist (LAMA). The corticosteroid can be one which is inhaled on a daily basis. An embodiment, a subject described herein, may be receiving a daily corticosteroid such as Fluticasone for example by inhalation, for example the subject may be receiving a corticosteroid which is TRELEGY or RELVAR. In one embodiment the subject may be receiving ≥ 440micrograms of Fluticasone propionate or another such agent in an amount that is clinically comparable according to the GINA 2020 guidelines. In some embodiments, a subject described herein is a subject who have been switched from a treatment comprising an agent targeting IL-5 or IL-5R to a treatment comprising an antigen binding protein described herein and wherein the subject is being treated with a nonbiologic treatment for asthma. In some embodiments, a subject described herein is a subject who have been switched from a treatment comprising an agent targeting IL-5 or IL-5R, for example mepolizumab, reslizumab, or benralizumab, to a treatment comprising an antigen binding protein described herein and wherein the subject is being treated with a nonbiologic treatment for asthma, for example corticosteroids and/or other controllers, such as long-acting beta-2-agonist (LABA) and/or long-acting muscarinic antagonist (LAMA). In some embodiments, the antigen binding protein is 28Y042-7F11-1.

In one embodiment the subject to be treated is receiving a maximum daily dose (µg) of corticosteroid as follows: Beclomethasone dipropionate (chorofluorocarbonate - CFC) = about 1000, Beclomethasone dipropionate (hydrofluoroalkane propellant - HFA) = about 400, Budesonide (DPI) = about 800, Ciclesonide (HFA) = about 320, Fluticasone propionate (dry powder inhaler - DPI) = about 500, Fluticasone furoate (DPI) = about 100, Mometasone furoate = about 440, Triamcinolone acetonide = about 2000.

As used herein, the phrase "once every 6 months" means that in a typical 6 month period consisting of about 183 days, a subject is administered a dose of the antigen binding protein of the disclosure on one day only and on the other days the subject is not administered a dose of the antigen binding protein of the disclosure. Administration of once every 6 months may also be referred to as "Q26W" (which refers to administration once every 26 weeks). References herein to "about once every 6 months" refer to an intended dosage regime of once every 6 months, but with the allowance of patient compliance, therefore allowing up to a four week variation depending upon patient scheduling.

Ranges provided herein, of any type, include all values within a particular range described and values about an endpoint for a particular range.

If desired, the effective dose of an antibody or antigen binding protein of the disclosure (e.g., as a pharmaceutical composition) may be administered as a unit dosage form. The administration of a dose may be by slow continuous infusion over a period of from 2 to 24 hours, such as from 2 to 12 hours, or from 2 to 6 hours. Such an administration may result in reduced side effects.

### ANTIGEN BINDING PROTEINS

The extended pharmacokinetic (PK) and pharmacology characteristics of the antigen binding protein described herein (28Y042-7F11-1) are achieved by reducing clearance and increasing affinity for IL-5 and are believed to enable a single administration every 26 weeks, as opposed to the current regimen of every 4 weeks for mepolizumab and reslizumab, or every 8 weeks for benralizumab (every 4 weeks for the first 3 doses).

### STATEMENT OF THE INVENTION

In one aspect, the present disclosure provides an antigen binding protein which binds to IL-5 and comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region, for use in the treatment of an IL-5 mediated disease such as asthma (for example mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma), by administration to a subject in an amount of about 100 mg to about 300 mg at a frequency of about once every 6 months. In one embodiment, the heavy chain variable region of the antigen binding protein further comprises a heavy chain FR4 amino acid sequence as shown in SEQ ID NO: 19.

Antigen binding proteins which bind to IL-5 and comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region and which are compounds of the disclosure and are disclosed in International Patent Application Publication Number WO2018215964.

In one embodiment, the antigen binding proteins of the disclosure are those which comprise a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region and which also further comprises a heavy chain FR4 amino acid sequence as shown in SEQ ID NO: 19.

In another embodiment the antigen binding proteins of the disclosure comprise: a heavy chain variable region sequence having the amino acid sequence shown in SEQ ID NO: 3; and a light chain variable region sequence having the amino acid sequence shown in SEQ ID NO: 4 and also a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region and also optionally a heavy chain FR4 amino acid sequence as shown in SEQ ID NO: 19.

In one embodiment the antigen binding protein of the disclosure is an antibody comprising a heavy chain and a light chain, wherein:
a) the heavy chain comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and
b) the light chain comprises a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10. The antibody can further comprise a heavy chain FR4 amino acid sequence as shown in SEQ ID NO: 19 and/or a heavy chain Fc domain having a tyrosine residue at position 252, a threonine residue at position 254 and a glutamic acid residue at position 256. The heavy chain Fc domain can be an IgG1 Fc domain, such as a human IgG1 Fc domain.

In another embodiment the antigen binding protein of the disclosure is an antibody comprising a heavy chain and a light chain, wherein:
a) the heavy chain comprises a heavy chain variable region sequence having the amino acid sequence shown in SEQ ID NO: 3; and
b) the light chain comprises a light chain variable region sequence having the amino acid sequence shown in SEQ ID NO: 4. The antibody can further comprise a heavy chain Fc domain having a tyrosine residue at position 252, a threonine residue at position 254 and a glutamic acid residue at position 256. The heavy chain Fc domain can be an IgG1 Fc domain, such as a human IgG1 Fc domain.

In another embodiment the antigen binding protein of the disclosure is an antibody comprising a heavy chain having the amino acid sequence shown in SEQ ID NO: 1 and a light chain having the amino acid sequence shown in SEQ ID NO: 2.

The antigen binding proteins of the disclosure can be variants of those described above which retain ability to bind to IL-5, for example useful variants can be those which bind to human IL-5 as determined at about 25 °C by KINEXA phase affinity analysis with a binding affinity of about at least 10pM or which bind to human IL-5 as determined at about 37 °C using BIACORE T200 with a binding affinity of about at least 30pM e.g. about 39pM. Useful variants can also have a binding affinity for human FcRn at pH 6.0 (as determined by BIACORE T200 at 37 °C) of about at least 150nM and at pH 7.4 (as determined by BIACORE T200 at 37 °C) of about at least 16100nM. Useful variants may also have an improved half life (PK) as determined in cynomolgus serum of about at least 20 days e.g. about 24 days. For example useful variants can include antibodies which have at least about 90% identity to any of the sequences described herein for example about 91%, 92%, 93%, 94%, 95%, 96%, 97%, 98%, 98.5%, 99%, 99.5%, 100% identity to any of the sequences described herein. For example the variant can be an antibody which comprises a heavy chain variable region sequence having at least about 90% identity t for example about 91%, 92%, 93%, 94%, 95%, 96%, 97%, 98%, 98.5%, 99%, 99.5%, 100% identity to the amino acid sequence shown in SEQ ID NO: 3; and/or a light chain variable region sequence having at least about 90% identity for example about 91%, 92%, 93%, 94%, 95%, 96%, 97%, 98%, 98.5%, 99%, 99.5%, 100% identity the amino acid sequence shown in SEQ ID NO: 4; the variant may further comprises a heavy chain FR4 amino acid sequence as shown in SEQ ID NO: 19 and may also further comprise a heavy chain Fc domain (such as an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region.

The variant can also be an antibody comprising a heavy chain having at least about 90% identity for example about 91%, 92%, 93%, 94%, 95%, 96%, 97%, 98%, 98.5%, 99%, 99.5%, 100% identity to the amino acid sequence shown in SEQ ID NO: 1 and a light chain having at least about 90% identity for example about 91%, 92%, 93%, 94%, 95%, 96%, 97%, 98%, 98.5%, 99%, 99.5%, 100% identity the amino acid sequence shown in SEQ ID NO: 2.

In an embodiment, the disclosure provides an antibody which binds to IL-5 and comprises a heavy chain having the amino acid sequence shown in SEQ ID NO: 1 and a light chain having the amino acid sequence shown in SEQ ID NO: 2, for use in the treatment of asthma, for example eosinophilic asthma (e.g. moderate or severe eosinophilic asthma), by administration to a human subject in an amount of about 100 mg to about 300 mg at a frequency of about once every 6 months.

28Y042-7F11-1 comprising the heavy chain amino acid sequence shown in SEQ ID NO: 1 and the light chain amino acid sequence shown in SEQ ID NO: 2 is an example of an antibody of the disclosure. 28Y042-7F11-1 or the antigen binding proteins of the disclosure bind human IL-5 and antagonizes its activity.

28Y042-7F11-1 is a recombinant humanized monoclonal antibody (IgG1, Kappa). 28Y042-7F11-1 has two light and two heavy chains.

The 28Y042-7F11-1 heavy chain variable region is encoded by the nucleic acid sequence shown in SEQ ID NO: 15. The 28Y042-7F11-1 light chain variable region is encoded by the nucleic acid sequence shown in SEQ ID NO: 16.

The 28Y042-7F11-1 full length heavy chain is encoded by the nucleic acid sequence shown in SEQ ID NO: 17. The 28Y042-7F11-1 full length light chain is encoded by the nucleic acid sequence shown in SEQ ID NO: 18. During manufacture, the nucleic acid sequence may comprise a leader sequence, therefore the 28Y042-7F11-1 full length heavy chain may be encoded by the nucleic acid sequence shown in SEQ ID NO: 13 and the 28Y042-7F11-1 full length light chain may be encoded by the nucleic acid sequence shown in SEQ ID NO: 14.

The 28Y042-7F11-1 heavy and light chains are covalently linked by a single disulfide bond and the heavy chains are linked to each other by two disulfide bonds resulting in a typical IgG molecule.

The antigen binding proteins described herein may be produced by any number of conventional techniques. For example, the antigen binding proteins may be expressed in and purified from recombinant expression systems. In one embodiment, the antigen binding protein is produced by a method of culturing a host cell under conditions suitable for expression of a polypeptide comprising SEQ ID NO: 1 and SEQ ID NO: 2, wherein the composition is expressed, and optionally purified, and optionally formulated within a pharmaceutical composition.

A number of different expression systems and purification regimes can be used to produce the compositions. Generally, host cells are transformed with a recombinant expression vector encoding the antibody. A wide range of host cells can be employed, including eukaryotic cell lines of mammalian origin (e.g., CHO, Perc6, HEK293, HeLa, NS0). Suitable host cells include mammalian cells such as CHO (e.g., CHOK1 and CHO-DG44).

The host cell may be an isolated host cell. The host cell is usually not part of a multicellular organism (e.g., plant or animal). The host cell may be a non-human host cell.

Appropriate cloning and expression vectors for use with eukaryotic or mammalian cellular hosts and methods of cloning are known in the art.

The cells may be cultured under conditions that promote expression of the antigen binding protein. For example, a production bioreactor is used to culture the cells. The production bioreactor volume may be: (i) about 20,000 litres, about 10,000 litres; about 5,000 litres; about 2,000 litres; about 1,000 litres; or about 500 litres; or (ii) between 500 and 20,000 litres; between 500 and 10,000 litres; between 500 and 5,000 litres; between 1,000 and 10,000 litres, or between 2,000 and 10,000 litres. For example, the cells may be cultured in a production bioreactor at a pH of about 6.75 to pH 7.00. Alternatively, the cells may be cultured in a production bioreactor for about 12 to about 18 days. Alternatively, the cells may be cultured in a production bioreactor at a pH of about 6.75 to pH 7.00, for about 12 to about 18 days. This culture step may help to control the level of deamidated antibody variants, for example, to reduce the level of deamidated antibody variants.

The antigen binding proteins may be recovered and purified by conventional protein purification procedures. For example, the antigen binding protein may be harvested directly from the culture medium. Harvest of the cell culture medium may be via clarification, for example by centrifugation and/or depth filtration. Recovery of the antigen binding protein is followed by purification to ensure adequate purity.

One or more chromatography steps may be used in purification, for example one or more chromatography resins; and/or one or more filtration steps. For example affinity chromatography using resins, such as protein A, G, or L may be used to purify the composition. Alternatively, or in addition to, an ion-exchange resin such as a cation-exchange may be used to purify the antigen binding protein. Alternatively, or in addition to, a hydrophobic interaction chromatographic resin may be used to purify the antigen binding protein. Alternatively the purification steps comprise: an affinity chromatography resin step, followed by a cation-exchange resin step, followed by a hydrophobic interaction chromatographic resin step.

For example, the harvest is placed in contact with a protein A resin. The solution comprising the composition may be eluted from the protein A resin and treated at pH 3.3 to 3.7 for 15 to 240 minutes. This protein A resin step may help to control the level of aggregated antibody variants, for example, to reduce the level of aggregated antibody variants.

The solution comprising the antigen binding protein may then be further clarified by depth filtration and/or dual layer filtration.

Alternatively, or in addition to, an anion exchange resin may be used. The solution comprising the antigen binding protein may be placed in contact with an anion exchange resin (for example Q-SEPHAROSE^{™} Fast Flow anion exchange chromatography) at a load pH of 8.3 to 8.7. The solution comprising the composition may be eluted from the anion exchange resin and held for 96 hours or less. This anion exchange resin step may help to control the level of deamidated antibody variants, for example, to reduce the level of deamidated antibody variants.

Optionally, guanidine and/or ammonium sulphate may be added to the solution comprising the composition, and held for 15 to 240 minutes.

Alternatively, or in addition to, a hydrophobic interaction chromatographic resin may be used. The solution comprising the antigen binding protein may be placed in contact with a hydrophobic interaction chromatographic resin (e.g., phenyl SEPHAROSE^{™} fast flow chromatography) at a load ratio of 12 to 27g protein /L resin. For example, the solution comprising the antigen binding protein may be eluted using an elution gradient volume (bed volumes; BV) of about 9 to about 11. An elution peak cut stop (% of maximum peak height) of about 17 to about 23 may be used during elution from the hydrophobic interaction chromatographic resin. This hydrophobic interaction chromatographic resin step may help to control the level of aggregated antibody variants, for example, to reduce the level of aggregated antibody variants.

### MEDICAL USE AND METHODS OF TREATMENT

The references to methods of treatment are to be interpreted as references to the compounds, pharmaceutical compositions and medicaments of the present disclosure for use in a method for treatment of the human (or animal) body by therapy (or for diagnosis).

The administration of an antigen binding protein which binds to IL-5 according to the disclosure in an amount of about 100 mg to about 300 mg (e.g., 100 mg) at a frequency of about once every 6 months can provide a safe and effective therapy for the treatment of an IL-5 mediated disease such as asthma (for example mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma). This administration strategy may also enhance efficacy as a result of increased patient compliance as patients only need to be dosed once every 6 months and thus this is a less burdensome regimen.

In one aspect, the present disclosure provides a method of treating an IL-5 mediated disease such as asthma (for example mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma), which method comprises administering to a subject in need thereof an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months.

In one embodiment, the IL-5 mediated disease is asthma. The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma. In a further embodiment, the asthma has an eosinophilic phenotype, e.g. is selected from mild eosinophilic asthma, moderate eosinophilic asthma or severe eosinophilic asthma. In one embodiment, the asthma is severe asthma.

The disclosure also provides a method of treating asthma (e.g. mild, moderate or severe asthma, such as mild, moderate or severe eosinophilic asthma) which method comprises administering to a human subject in need thereof an antigen binding protein which binds to IL-5, the antigen binding protein comprising a heavy chain variable region sequence having the amino acid sequence shown in SEQ ID NO: 3 and a light chain variable region sequence having the amino acid sequence shown in SEQ ID NO: 4 in an amount of about 100 mg to about 300 mg, about once every 6 months.

The disclosure also provides a method of treating asthma (e.g. mild, moderate or severe asthma, such as mild, moderate or severe eosinophilic asthma) which method comprises administering to a human subject in need thereof an antibody which binds to IL-5, the antigen binding protein comprising a heavy chain having the amino acid sequence shown in SEQ ID NO: 1 and a light chain having the amino acid sequence shown in SEQ ID NO: 2 in an amount of about 100 mg to about 300 mg, about once every 6 months.

In one aspect, the present disclosure provides a method of treating an IL-5 mediated disease in a subject, the method comprising: a) identifying a subject with a disease selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma; and b) administering about 100 mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, to the subject about once every 6 months; whereby the disease in the subject is treated.

In an embodiment the IL-5 mediated disease is selected from: Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) and dermatitis. The disease may be characterised by an eosinophilc phenotype. Inflammatory bowel disease can be Crohns disease or Ulcerative colitis.

In an embodiment, there is provided use of an antigen binding protein as described herein for the treatment of chronic rhinosinusitis with nasal polyps (CRSwNP) by administration to a subject about once every 6 months, wherein the antigen binding protein is in an amount of about 100 mg.

Subjects to be treated with CRSwNP may have severe CRSwNP and/or be receiving SOC therapy for example treatment with intranasal corticosteroids. Therapy with corticosteroids and/or surgery may not provide adequate disease control.

In an embodiment, there is provided use of an antigen binding protein as described herein for the treatment of eosinophilic granulomatosis with polyangiitis (EGPA) by administration to a subject about once every 6 months, wherein the antigen binding protein is in an amount of about 200 mg.

Subjects to be treated with EGPA may have relapsing-remitting or refractory eosinophilic granulomatosis with polyangiitis. Treatment according to the disclosure may be in addition to other SOC treatment.

In an embodiment, there is provided use of an antigen binding protein as described herein for the treatment of hypereosinophilic syndrome (HES) by administration to a subject about once every 6 months, wherein the antigen binding protein is in an amount of about 200 mg.

Subjects to be treated with HES may have inadequately controlled hypereosinophilic syndrome without an identifiable non-haematologic secondary cause. Treatment according to the disclosure may be in addition to other SOC treatment.

When the dose is 200 mg, this may be contained in one unit or in multiple units (e.g. 2 units each comprising 100 mg of active ingredient)

Subjects to be treated can be paediatric or adult human subjects. However when the subject to be treated weighs less than 40Kg, such as a paediatric patient, the dose can be reduced e.g. the 200mg dose every 6 months for HES and EGPA can be reduced e.g. 100mg every 6 months can be given.

In one aspect, the present disclosure provides a method of treating an IL-5 mediated disease in a subject, the method comprising administering about 100 mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, to the subject about once every 6 months; whereby the disease in the subject is treated.

As described herein, the heavy chain variable region of the antigen binding protein may further comprise a heavy chain FR4 amino acid sequence as shown in SEQ ID NO: 19. In one embodiment, the heavy chain Fc domain is an IgG1 Fc domain. In a further embodiment, the heavy chain Fc domain is a human IgG1 Fc domain.

In one embodiment, the antigen binding protein comprises: a heavy chain variable region sequence having the amino acid sequence shown in SEQ ID NO: 3; and a light chain variable region sequence having the amino acid sequence shown in SEQ ID NO: 4. In a further embodiment, the antigen binding protein is an antibody comprising a heavy chain having the amino acid sequence shown in SEQ ID NO: 1 and a light chain having the amino acid sequence shown in SEQ ID NO: 2.

In one embodiment, the method comprises administering about 100 mg of the antigen binding protein.

In one embodiment, the method comprises administering the antigen binding protein subcutaneously.

In one embodiment, the subject is a human subject.

Preferably, the subject, for example a patient with asthma (e.g. mild, moderate or severe asthma, such as mild, moderate or severe eosinophilic asthma), prior to treatment, for example at any point in the 12 months prior to treatment has an absolute blood eosinophil count selected from the group consisting greater than or equal to 100 cells per µL, such as greater than or equal to 150 cells per µL, or greater than or equal to 200 cells per µL or greater than or equal to 250 cells per µL , or greater than or equal to 300 cells per µL, or greater than or equal to 350 cells per µL.

In one embodiment, there is provided use of an antigen binding protein as described herein in the manufacture of a medicament for the treatment of an IL-5 mediated disease for administration about once every 6 months, wherein the medicament comprises the antigen binding protein in an amount of about 100 mg to about 300 mg.

In another aspect, the present disclosure provides a method of decreasing an absolute blood eosinophil count in a subject, the method comprising: a) identifying a subject having a condition selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma; and b) administering to the subject an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months, whereby the absolute blood eosinophil count in the subject is decreased.

The disclosure also provides a method of decreasing an absolute blood eosinophil count in a subject, the method comprising: a) identifying a subject having asthma (e.g. mild, moderate or severe asthma, such as mild, moderate or severe eosinophilic asthma) and b) administering to said subject an antigen binding protein which binds to IL-5, the antigen binding protein comprising: a heavy chain variable region sequence having the amino acid sequence shown in SEQ ID NO: 3; and a light chain variable region sequence having the amino acid sequence shown in SEQ ID NO: 4, in an amount of about 100 mg to about 300 mg, about once every 6 months, whereby the absolute blood eosinophil count in the subject is decreased.

The disclosure also provides a method of decreasing an absolute blood eosinophil count in a subject, the method comprising: a) identifying a subject having asthma (e.g. mild, moderate or severe asthma, such as mild, moderate or severe eosinophilic asthma) and b) administering to said subject an antibody which binds to IL-5, the antigen binding protein comprising a heavy chain having the amino acid sequence shown in SEQ ID NO: 1 and a light chain having the amino acid sequence shown in SEQ ID NO: 2, in an amount of about 100 mg to about 300 mg, about once every 6 months, whereby the absolute blood eosinophil count in the subject is decreased.

One embodiment of the method of the disclosure further comprises: a) making a first measurement of an absolute blood eosinophil count in the subject; b) making a second measurement of an absolute blood eosinophil count in the subject after administering to the subject a therapeutically effective amount of the antigen binding protein; and c) comparing the first measurement and second measurement to determine effectiveness of therapy.

One embodiment of the method of the disclosure further comprises : a) making a first measurement of an absolute blood eosinophil count in the subject; b) making a second measurement of an absolute blood eosinophil count in the subject after administering to the subject a therapeutically effective amount of the antigen binding protein; and c) comparing the first measurement and second measurement; wherein the subject has an absolute blood eosinophil count selected from the group consisting of greater than or equal to 150 cells per µL, greater than or equal to 200 cells per µL and greater than or equal to 350 cells per µL.

In one embodiment, the method comprises administering about 100 mg of the antigen binding protein.

In one embodiment, the method comprises administering the antigen binding protein subcutaneously.

In one embodiment, the subject, such as a patient with asthma (e.g. mild, moderate or severe asthma, such as mild, moderate or severe eosinophilic asthma) (at screening at the start of treatment) has an absolute blood eosinophil count selected from the group consisting of greater than or equal to 150 cells per µL, such as greater than or equal to 200 cells per µL. In another embodiment the subject such as a patient with asthma (e.g. mild, moderate or severe asthma, such as mild, moderate or severe eosinophilic asthma) has greater than or equal to 300 eosinophils per uL of blood in the past 12 months prior to treatment.

In some embodiments, described herein is a method of reducing absolute blood eosinophil count, the method comprising administering an antigen binding protein which binds to IL-5 to a subject, wherein absolute blood eosinophil count is maintained or decreased compared to baseline prior to treatment. In some embodiments, the subject is a mammal. In some embodiments, the subject is human. In some embodiments, described herein is a method for treating asthma, the method comprising administering an antigen binding protein which binds to IL-5 to a subject, wherein absolute blood eosinophil count is maintained or decreased compared to baseline prior to treatment. In some embodiments at least about 1, 2, 3, 4, 5, 8, 10, 12, 15, 18, 20, 24, 26, 30, 32, 36, 40, or 50 weeks following a single administration of the antigen binding protein which binds to IL-5 to the subject, the blood eosinophil count is reduced ≥ 50%, 55%, 60%, 65%, 70%, 75%, 80%, 85%, 90%, 95% compared to baseline prior to administration. In some embodiments, at least about 26 weeks following a single administration of the antigen binding protein to the subject, the absolute blood eosinophil count is reduced ≥ 50%, 55%, 60%, 65%, 70%, 75%, 80% compared to baseline prior to administration. In some embodiments, at least about 32 weeks following a single administration of the antigen binding protein to the subject, the absolute blood eosinophil count is reduced ≥ 50%, 55%, 60%, 65%, 70%, 75%, 80% compared to baseline prior to administration. In some embodiments, at least about 36 weeks following a single administration of the antigen binding protein to the subject, the absolute blood eosinophil count is reduced ≥ 50%, 55%, 60%, 65%, 70%, 75%, 80% compared to baseline prior to administration. In some embodiments, at least about 40 weeks following a single administration of the antigen binding protein to the subject, the absolute blood eosinophil count is reduced ≥ 50%, 55%, 60%, 65%, 70%, 75%, 80% compared to baseline prior to administration. In some embodiments, 100 mg of the antigen binding protein which binds to IL-5 is administered to the subject and 24 weeks following a single administration of the antigen binding protein to the subject, the absolute blood eosinophil count is reduced by at least about 80% compared to baseline prior to administration. In some embodiments, 100 mg of the antigen binding protein which binds to IL-5 is administered to the subject and 26 weeks following a single administration of the antigen binding protein to the subject, the absolute blood eosinophil count is reduced by at least about 82% compared to baseline prior to administration. In some embodiments, 300 mg of the antigen binding protein which binds to IL-5 is administered to the subject and 26 weeks following a single administration of the antigen binding protein to the subject, the absolute blood eosinophil count is reduced by at least about 83% compared to baseline prior to administration. In some embodiments, 300 mg of the antigen binding protein which binds to IL-5 is administered to the subject and 24 weeks following a single administration of the antigen binding protein to the subject, the absolute blood eosinophil count is reduced by at least about 80% compared to baseline prior to administration. In some embodiments, the antigen binding protein which binds to IL-5 comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7. In some embodiments, the antigen binding protein which binds to IL-5 comprises a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10. In some embodiments, the antigen binding protein which binds to IL-5 comprises a heavy chain Fc domain having a tyrosine residue at position 252, a threonine residue at position 254 and a glutamic acid residue at position 256. In some embodiments, the antigen binding protein which binds to IL-5 comprises an amino terminus of the heavy chain Fc domain connected to a carboxy terminus of the heavy chain variable region. In some embodiments, the antigen binding protein which binds to IL-5 comprises a heavy chain having the amino acid sequence shown in SEQ ID NO: 1 and a light chain having the amino acid sequence shown in SEQ ID NO: 2. In some embodiments, the asthma comprises mild asthma, moderate asthma, severe asthma, mild eosinophilic asthma, moderate eosinophilic asthma, severe eosinophilic asthma, uncontrolled eosinophilic asthma, eosinophilic asthma or sub-eosinophilic asthma. In some embodiments, the asthma comprises severe asthma, such as severe eosinophilic asthma.

The dosage regimens described herein are for use in the treatment of IL-5 mediated diseases, particularly asthma, in a human or animal subject. In one embodiment, the subject is a human.

The asthma may be selected from: mild asthma, moderate asthma, severe asthma, eosinophilic asthma, mild eosinophilic asthma, moderate eosinophilic asthma, severe eosinophilic asthma, uncontrolled eosinophilic asthma, eosinophilic asthma or sub-eosinophilic asthma. In one embodiment, the asthma is mild or moderate asthma. In one embodiment, the asthma is severe asthma. The disclosure finds particular use in subjects with asthma with an eosinophilic phenotype. Therefore, in a further embodiment, the asthma is mild, moderate or severe eosinophilic asthma.

### PHARMACEUTICAL COMPOSITIONS

It is common to present the antigen binding protein as a pharmaceutical composition. In one embodiment, the pharmaceutical composition comprising an antigen binding protein of the disclosure further comprises a pharmaceutically acceptable excipient. In a further embodiment, the pharmaceutical composition comprises an antigen binding protein of the disclosure and one or more pharmaceutically acceptable excipient(s).

In one aspect, the present disclosure provides a pharmaceutical composition comprising from about 100 mg to about 300 mg of an antigen binding protein which binds to IL-5, wherein the antigen binding protein comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, and a pharmaceutically acceptable excipient.

In one embodiment, the pharmaceutical composition comprises an antigen binding protein of the disclosure in an amount of about 100 mg, and a pharmaceutically acceptable excipient.

In one embodiment, the pharmaceutical composition comprises an antigen binding protein of the disclosure wherein the heavy chain variable region of the antigen binding protein further comprises a heavy chain FR4 amino acid sequence as shown in SEQ ID NO: 19.

In one embodiment, the pharmaceutical composition comprises an antigen binding protein of the disclosure wherein the heavy chain Fc domain is an IgG1 Fc domain. The heavy chain Fc domain may be a human IgG1 Fc domain.

In one embodiment, the pharmaceutical composition comprises an antigen binding protein of the disclosure wherein the antigen binding protein comprises: a heavy chain variable region sequence having the amino acid sequence shown in SEQ ID NO: 3; and a light chain variable region sequence having the amino acid sequence shown in SEQ ID NO: 4.

The disclosure also provides a pharmaceutical composition comprising an antigen binding protein which binds to IL-5, the antigen binding protein comprising a heavy chain variable region sequence having the amino acid sequence shown in SEQ ID NO: 3 and a light chain variable region sequence having the amino acid sequence shown in SEQ ID NO: 4, in an amount of about 100 mg, and a pharmaceutically acceptable excipient.

In one embodiment, the pharmaceutical composition comprises an antigen binding protein of the disclosure wherein the antigen binding protein is an antibody comprising a heavy chain having the amino acid sequence shown in SEQ ID NO: 1 and a light chain having the amino acid sequence shown in SEQ ID NO: 2.

The disclosure also provides a pharmaceutical composition comprising an antibody which binds to IL-5, the antigen binding protein comprising a heavy chain having the amino acid sequence shown in SEQ ID NO: 1 and a light chain having the amino acid sequence shown in SEQ ID NO: 2 in an amount of about 100 mg, and a pharmaceutically acceptable excipient.

In one embodiment, the pharmaceutical composition is formulated for subcutaneous administration. In particular, the pharmaceutical composition may be formulated for administration to a human subject. The subject (prior to treatment) may have an absolute blood eosinophil count selected from the group consisting of greater than or equal to 150 cells per µL, such as greater than or equal to 200 cells per µL. The subject (at screening at the start of treatment or prior to start of treatment) may have an absolute blood eosinophil count selected from the group consisting of greater than or equal to 150 cells per µL, such as greater than or equal to 200 cells per µL and/or the subject may have greater than or equal to 300 eosinophils per uL of blood in the past 12 months prior to treatment.

In one embodiment, the pharmaceutical composition is for administration to a subject about once every 3 months, once every 4 months, once every 5 months or once every 6 months. In a further embodiment, the pharmaceutical composition is for administration to a subject about once every 6 months.

In one embodiment, the pharmaceutical composition is administered at an administration interval (or treatment cycle) of about once every 13 weeks (Q13W), once every 17 weeks (Q17W), once every 22 weeks (Q22W) or once every 26 weeks (Q26W). In a further embodiment, the pharmaceutical composition is for administration to a subject about once every 26 weeks (Q26W).

In a further aspect, the present disclosure provides a pharmaceutical composition for use in the treatment of an IL-5 mediated disease such as asthma (for example mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma) wherein the composition comprises from about 100 mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain (e.g. an IgG1 Fc) having a tyrosine residue at position 252, a threonine residue at position 254 and a glutamic acid residue at position 256 and wherein an amino terminus of the heavy chain Fc domain is connected to a carboxy terminus of the heavy chain variable region, wherein the pharmaceutical composition is for administration to a subject about once every 6 months. In one embodiment of this heavy chain variable region of the antigen binding protein further comprises a heavy chain FR4 amino acid sequence as shown in SEQ ID NO: 19.

The disclosure also provides a pharmaceutical composition for use in the treatment of asthma (e.g. mild, moderate or severe asthma, such as mild, moderate or severe eosinophilic asthma) wherein the composition comprises about 100 mg to about 300 mg of an antigen binding protein which binds to IL-5, the antigen binding protein comprising: a heavy chain variable region sequence having the amino acid sequence shown in SEQ ID NO: 3; and a light chain variable region sequence having the amino acid sequence shown in SEQ ID NO: 4, wherein the pharmaceutical composition is for administration to a human subject about once every 6 months.

The disclosure also provides a pharmaceutical composition for use in the treatment of asthma (e.g. mild, moderate or severe asthma, such as mild, moderate or severe eosinophilic asthma) wherein the composition comprises about 100 mg to about 300 mg of an antibody which binds to IL-5, the antigen binding protein comprising a heavy chain having the amino acid sequence shown in SEQ ID NO: 1 and a light chain having the amino acid sequence shown in SEQ ID NO: 2, wherein the pharmaceutical composition is for administration to a human subject about once every 6 months.

28Y042-7F11-1 or the antigen binding proteins of the disclosure can be provided as a lyophilized powder containing the antibody and excipients which can be reconstituted with a pharmaceutically acceptable carrier (e.g., sterile water). This reconstituted pharmaceutical composition can then be administered either subcutaneously or intravenously (e.g., with further dilution). 28Y042-7F11-1 or the antigen binding proteins of the disclosure can also be provided as a liquid formulation containing the antibody, excipients and a pharmaceutically acceptable carrier. This liquid pharmaceutical composition can then be administered either subcutaneously or intravenously (e.g., with further dilution).

The pharmaceutical compositions described herein may comprise purified preparations of an antigen binding protein as described herein. For example, the pharmaceutical preparation may comprise a purified preparation of an antigen binding protein as described herein in combination with a pharmaceutically acceptable carrier.

Typically, such pharmaceutical compositions comprise a pharmaceutically acceptable carrier as known and called for by acceptable pharmaceutical practice. Examples of such carriers include sterilized carriers, such as saline, Ringers solution, or dextrose solution, optionally buffered with suitable buffers to a pH within a range of 5 to 8.

Pharmaceutical compositions may be administered by injection or infusion (e.g., intravenous, intraperitoneal, intradermal, subcutaneous, intramuscular, or intraportal, in particular subcutaneous). Such compositions are suitably free of visible particulate matter.

Formulations described herein are stable pharmaceutical compositions.

Preferably, the pharmaceutical composition comprises an aqueous liquid formulation. The pharmaceutical composition may be within a pH range of 5 to 8. For example, the pharmaceutical composition may be within a pH range of between 5.5 to 7.5, such as between 5.8 to 7.2, 5.9 to 6.7, or 6.0 to 6.5. In particular, the pharmaceutical composition is at about pH 6.0. In some embodiments, the pharmaceutical composition is at about pH 5.6, 5.7, 5.8, 5.9, 6.0, 6.1, 6.2, 6.3 6.4, 6.5 or 6.6

Methods for the preparation of such pharmaceutical compositions are well known to those skilled in the art. Pharmaceutical compositions may comprise the antigen binding protein in unit dosage form, optionally together with instructions for use. Pharmaceutical compositions may be lyophilized (freeze dried) for reconstitution prior to administration according to methods well known or apparent to those skilled in the art. Where antibodies have an IgG1 isotype, a chelator of copper, such as citrate (e.g., sodium citrate) or EDTA or histidine, may be added to the pharmaceutical composition to reduce the degree of copper-mediated degradation of antibodies of this isotype. Pharmaceutical compositions may also comprise a solubilizer, such as arginine, a surfactant/anti-aggregation agent such as polysorbate 80, and an inert gas such as nitrogen to replace vial headspace oxygen.

The compositions of the disclosure may further comprise a buffering agent selected from the group consisting of histidine, sodium phosphate dibasic heptahydrate, phosphate, citric acid, citrate, sodium phosphate, potassium phosphate and sodium citrate, providing a pH of between 5.8 and 7.2 or a pH of from pH 6.0 to pH 6.6, with a pH value of 6.0 being preferred. The buffer in the compositions of the disclosure may be present in the range from about 10-30 mM, about 10-20 mM, about 20 mM or about 15.5 mM. In one embodiment, the composition comprises histidine in a range from about 5-50mM, such as about 10-50 mM, about 10-30 mM or about 10-20 mM. For example, the buffer in the compositions of the disclosure is present at about 20 mM histidine.

In some embodiments, a composition comprises a surfactant. "Surfactants" are surface active agents that can exert their effect at surfaces of solid-solid, solid-liquid, liquid-liquid, and liquid-air interfaces because of their chemical composition, containing both hydrophilic and hydrophobic groups. Surfactants may reduce the concentration of proteins in dilute solutions at the air-water and/or water-solid interfaces where proteins can be adsorbed and potentially aggregated. Surfactants can bind to hydrophobic interfaces in protein formulations. Some parentally acceptable non-ionic surfactants comprise either polysorbate or polyether groups. Polysorbate 20 and 80, in particular polysorbate 80 (PS80), are suitable surfactant stabilizers in compositions of the disclosure. Therefore, the compositions of the disclosure may further comprise polysorbate 80. Polysorbate 80 may be present in the range from about 0.01-0.1% weight by volume, such as about 0.01% to about 0.05% or about 0.01 to about 0.03% w/v. For example, polysorbate 80 may be present in the compositions of the disclosure at about 0.02% weight by volume (0.02% w/v).

The compositions of the disclosure may further comprise EDTA which may contribute to stability of the formulation. EDTA may be present in the range from about 0.01-0.1 mM, such as about 0.02-0.08 mM or about 0.03-0.06 mM. For example, EDTA may be present at about 0.05 mM. The compositions of the disclosure may comprise 20-60 mM Histidine. The compositions of the disclosure may comprise 30-110 mM Arginine. The compositions of the disclosure may comprise 0-30 mM Methionine. The compositions of the disclosure may comprise 0-110 mM Glycine. The compositions of the disclosure may comprise 0-350 mM Trehalose.

The compositions of the disclosure may further comprise a sugar. In some embodiments, the composition comprises a polyol. In some embodiments, the polyol is a sugar, and preferably a non-reducing sugar. In some embodiments, the non-reducing sugar is trehalose. Therefore, the compositions of the disclosure may further comprise trehalose. In some embodiments, the composition comprises trehalose in the range from about from about 100 mM to about 250 mM, such as about 150 mM to about 200 mM, in particular about 160 mM to about 190 mM. For example, the trehalose may be present at about 180 mM.

The composition may also comprise a solubilizer, such as arginine, for example L-Arginine-HCl. In some embodiments, the composition comprises arginine in the range from about from about 10 mM to about 120 mM, such as about 20 mM to about 100 mM, in particular about 30 mM to about 50 mM. In some embodiments, the composition comprises about 40 mM arginine.

In one embodiment, the pharmaceutical composition comprises one or more pharmaceutically acceptable excipients selected from: histidine, trehalose, arginine, EDTA and polysorbate 80. In a further embodiment, the pharmaceutical composition comprises histidine, trehalose, arginine, EDTA and polysorbate 80. In some embodiments, the pharmaceutical composition comprises glycine.

In one embodiment, the pharmaceutical composition comprises about 20 mM histidine, about 180 mM trehalose, about 40 mM arginine, about 0.05 mM EDTA and about 0.02% weight of polysorbate 80 to volume.

As would be expected, any excipients utilised are pharmaceutically acceptable and be compatible with the other components (other excipients and the active ingredient) of the composition. In accordance with another aspect of the disclosure there is also provided a process for the preparation of a pharmaceutical composition including admixing an antigen binding protein of the disclosure, in an amount of about 100 mg to about 300 mg (preferably 100 mg), with a pharmaceutically acceptable excipient.

Pharmaceutical compositions for use in the treatment of an IL-5 mediated disease according to the present disclosure may be administered subcutaneously. Such compositions may be prepared by any method known in the art of pharmacy, for example by bringing into association the active ingredient with the excipient(s).

In one embodiment, the present disclosure provides an antigen binding protein disclosed herein, for use in the treatment of an IL-5 mediated disease, such as asthma, in a human or animal subject, by subcutaneous administration in an amount of about 100 mg to about 300 mg at a frequency of about once every six months.

In a further aspect the present disclosure provides an antigen binding protein disclosed herein, for use in the treatment of an IL-5 mediated disease, such as asthma, in a human or animal subject, by subcutaneous administration in an amount of about 30 mg to about 300 mg, or about 30 mg to about 200mg, at a frequency of about once every six months, for example the amount administered can be about 30mg to about 100mg, such as 40 mg or above, 50mg or above, 60 mg or above 70 mg or above, 80 mg or above 90mg or above, administered every 6 months.

Pharmaceutical compositions may be presented in unit dose forms containing a predetermined amount of active ingredient per unit dose. Preferred unit dosage compositions are those containing the dose for an entire day or sub-dose, or an appropriate fraction thereof, of an active ingredient. Unit doses that contain a sub-dose of the recommended dose for a day may therefore be administered more than once in order to make up the total dose for a day. For example, if a dose on a particular day is 100 mg, this may be contained in one unit or in multiple units (e.g. 2 units each comprising 50 mg of active ingredient). In one embodiment, the pharmaceutical composition is in unit dosage form.

### PRE-FILLED SYRINGES

The present disclosure provides a pre-filled syringe comprising about 100mg to about 300 mg of an antigen binding protein of the disclosure. In one embodiment, there is provided a pre-filled syringe comprising a pharmaceutical composition as disclosed herein.

In one aspect, the disclosure provides a pre-filled syringe comprising: a) about 100mg to about 300 mg (e.g., 100 mg) of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region; and b) a pharmaceutically acceptable excipient.

In a further aspect the present disclosure provides provides a pre-filled syringe comprising about 30mg to about 300 mg of an antigen binding protein of the disclosure, or about 30 mg to about 200mg, for example the prefilled syringe can comprise about 30mg to about 100mg of the antigen binding protein such as 40 mg or above, 50mg or above, 60 mg or above, 70 mg or above, 80 mg or above, 90mg or above.

In one embodiment, there is provided a pre-filled syringe comprising a pharmaceutical composition as disclosed herein.

In one embodiment, the heavy chain variable region of the antigen binding protein further comprises a heavy chain FR4 amino acid sequence as shown in SEQ ID NO: 19. In another embodiment, the heavy chain Fc domain is an IgG1 Fc domain. In a further embodiment, the heavy chain Fc domain is a human IgG1 Fc domain.

In one embodiment, the antigen binding protein comprises: a heavy chain variable region sequence having the amino acid sequence shown in SEQ ID NO: 3; and a light chain variable region sequence having the amino acid sequence shown in SEQ ID NO: 4. Therefore, the disclosure also provides a pre-filled syringe comprising about 100mg of an antigen binding protein which binds to IL-5 comprising: a heavy chain having a heavy chain variable region sequence having the amino acid sequence shown in SEQ ID NO: 3; and a light chain variable region sequence having the amino acid sequence shown in SEQ ID NO: 4, and a pharmaceutically acceptable excipient.

In one embodiment, the antigen binding protein is an antibody comprising a heavy chain having the amino acid sequence shown in SEQ ID NO: 1 and a light chain having the amino acid sequence shown in SEQ ID NO: 2. Therefore, the disclosure also provides a pre-filled syringe comprising about 100 mg of an antibody which binds to IL-5, the antigen binding protein comprising a heavy chain having the amino acid sequence shown in SEQ ID NO: 1 and a light chain having the amino acid sequence shown in SEQ ID NO: 2, and a pharmaceutically acceptable excipient.

In one embodiment, the pre-filled syringe is for subcutaneous administration. In a further embodiment, the pre-filled syringe is administered to a human subject. The subject (prior to treatment) may have an absolute blood eosinophil count selected from the group consisting of greater than or equal to 150 cells per µL, such as greater than or equal to 200 cells per µL.

In one embodiment, the pre-filled syringe is administered about once every 6 months. In one embodiment, the pre-filled syringe is administered about once every 26 weeks (Q26W).

In one embodiment, the pre-filled syringe comprises an aqueous liquid formulation. Formulations may also comprise a solubilizer, such as arginine, a surfactant/anti-aggregation agent such as polysorbate 80, polyol such as a sugar, and/or buffering agent as described herein.

The pre-filled syringe may comprise a buffering agent selected from the group consisting of histidine, sodium phosphate dibasic heptahydrate, phosphate, citric acid, citrate, sodium phosphate, potassium phosphate and sodium citrate, providing a pH of between 5.8 and 7.2 or a pH of from pH 6.0 to pH 6.6, with a pH value of 6.0 being preferred. The buffer may be present in the range from about 10-30 mM, about 10-20 mM, about 20 mM or about 15.5 mM. In one embodiment, the formulation present in the pre-filled syringe comprises histidine in a range from about 5-50mM, such as about 10-50 mM, about 10-30 mM or about 10-20 mM. For example, the buffer is present at about 20 mM histidine.

In some embodiments, the pre-filled syringe comprises a surfactant. Polysorbate 20 and 80, in particular polysorbate 80 (PS80), are suitable surfactant stabilizers in compositions of the disclosure. Therefore, the formulation present in the pre-filled syringe may further comprise polysorbate 80. Polysorbate 80 may be present in the range from about 0.01-0.1% weight by volume, such as about 0.01% to about 0.05% or about 0.01 to about 0.03% w/v. For example, polysorbate 80 may be present in the formulation at about 0.02% weight by volume (0.02% w/v).

The pre-filled syringe may further comprise EDTA. EDTA may be present in the range from about 0.01-0.1 mM, such as about 0.02-0.08 mM or about 0.03-0.06 mM. For example, EDTA may be present at about 0.05 mM.

The pre-filled syringe may further comprise a sugar. In some embodiments, the formulation present in the pre-filled syringe comprises a polyol. In some embodiments, the polyol is a sugar, and preferably a non-reducing sugar. In some embodiments, the non-reducing sugar is trehalose. Therefore, the formulation present in the pre-filled syringe may further comprise trehalose. In some embodiments, the formulation present in the pre-filled syringe comprises trehalose in the range from about from about 100 mM to about 250 mM, such as about 150 mM to about 200 mM, in particular about 160 mM to about 190 mM. For example, the trehalose may be present at about 180 mM.

The pre-filled syringe may also comprise a solubilizer, such as arginine, for example L-Arginine-HCI. In some embodiments, the formulation present in the pre-filled syringe comprises arginine in the range from about from about 10 mM to about 120 mM, such as about 20 mM to about 100 mM, in particular about 30 mM to about 50 mM. In some embodiments, the composition comprises about 40 mM arginine.

In particular, the aqueous liquid formulation is at about pH 6.0. In particular, the liquid formulation is at about pH 5.6, 5.7, 5.8, 5.9, 6.0, 6.1, 6.2, 6.3 6.4, 6.5 or 6.6. The formulation preferably contains the antigen binding protein and one or more pharmaceutically acceptable excipients. The excipients may be selected from one or more of: histidine, trehalose, arginine, EDTA and polysorbate 80. In a further embodiment, the excipients comprise histidine, trehalose, arginine, EDTA and polysorbate 80. In a further embodiment, pre-filled syringe comprises an aqueous liquid formulation at about pH 6.0 containing the antigen binding protein and about 20 mM histidine, about 180 mM trehalose, about 40 mM arginine, about 0.05 mM EDTA and about 0.02% weight of polysorbate 80 to volume.

In some embodiments, the pre-filled syringe may comprise EDTA present in the range from about 0.01-0.1 mM, such as about 0.02-0.08 mM or about 0.03-0.06 mM. For example, EDTA may be present at about 0.05 mM. In some embodiments, the pre-filled syringe may 20-60 mM Histidine. In some embodiments, the pre-filled syringe may comprise 30-110 mM Arginine In some embodiments, the pre-filled syringe may comprise 0-30 mM Methionine. In some embodiments, the pre-filled syringe may comprise 0-110 mM Glycine. In some embodiments, the pre-filled syringe may comprise 0-350 mM Trehalose.

In one aspect, the disclosure provides a pre-filled syringe as disclosed herein for use in the treatment of an IL-5 mediated disease. The IL-5 mediated disease may be asthma. In particular, the asthma is selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma. The asthma may be mild or moderate asthma, such as mild or moderate eosinophilic asthma. The asthma may be severe asthma, such as severe eosinophilic asthma.

In one embodiment, the pre-filled syringe is provided in a safety syringe device (SSD) or an autoinjector. Such devices are well known to a person skilled in the art.

### COMBINATION THERAPY

In certain embodiments, the antigen binding proteins according to the present disclosure may be administered in combination with one or more (e.g. two) other therapeutic agents that treat an IL-5 mediated disease, such as asthma.

These additional therapeutic agents may be administered via the same or a different administration route. Furthermore, they may be administered in the same dosage/dosage regimen as the antigen binding proteins or pharmaceutical compositions according to the present disclosure.

If additional therapeutic agents are utilised, in one embodiment, the combined action of the antigen binding protein and the additional therapeutic agent(s) is greater than the sum or each acting separately. The combination of two or more therapeutic agents may, thus, provide a synergistic effect.

Combination therapy with one or more additional therapeutic agents includes coadministration (simultaneous administration) and sequential or consecutive administration. Agents intended for use in combination may be formulated in separate compositions or, if the same dosage regimen can be used for all agents, a single pharmaceutical composition.

### CLAUSES

In summary, the disclosure includes:
A pharmaceutical composition comprising from about 100 mg to about 300 mg of an antigen binding protein which binds to IL-5, wherein the antigen binding protein comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, and a pharmaceutically acceptable excipient.

The pharmaceutical composition may comprise about 100 mg of the antigen binding protein.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The pharmaceutical composition may be for subcutaneous administration.

The pharmaceutical composition may be administered about once every 6 months.

The pharmaceutical composition may be administered about once every 26 weeks (Q26W).

The pharmaceutical composition may be administered to a human subject.

The human subject may have an absolute blood eosinophil count greater than or equal to 200 cells per µL.

The pharmaceutical composition may comprise an aqueous liquid formulation at about pH 6.0 containing the antigen binding protein and histidine, trehalose, arginine, EDTA and/or polysorbate 80.

The pharmaceutical composition may comprise an aqueous liquid formulation at about pH 6.0 containing the antigen binding protein and about 20 mM histidine, about 180 mM trehalose, about 40 mM arginine, about 0.05 mM EDTA and about 0.02% weight of polysorbate 80 to volume.

The pharmaceutical composition may be for use in the treatment of an IL-5 mediated disease.

The IL-5 mediated disease may be asthma, Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) or dermatitis.

The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma.

The asthma may be severe asthma, such as severe eosinophilic asthma.

An antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, for use in the treatment of an IL-5 mediated disease, by administration to a subject at a dose of about 100 mg to about 300 mg, at a frequency of about once every 6 months.

The dose may be about 100 mg.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The antigen binding protein may be administered to the subject subcutaneously.

The antigen binding protein may be administered to a human subject such as a adult or paediatric human subject.

The human subject may have an absolute blood eosinophil count greater than or equal to 200 cells per µL.

The IL-5 mediated disease may be selected from: asthma, Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) or dermatitis.

The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma.

The asthma may be severe asthma, such as severe eosinophilic asthma.

A pharmaceutical composition for use in the treatment of an IL-5 mediated disease wherein the composition comprises from about 100 mg to about 300 mg of an antigen binding protein which binds to IL-5, wherein the antigen binding protein comprises a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, and wherein the pharmaceutical composition is for administration to a subject about once every 6 months.

The pharmaceutical composition may comprise about 100 mg of the antigen binding protein.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The pharmaceutical composition may be for subcutaneous administration.

The pharmaceutical composition may be administered to a human subject.

The human subject may have an absolute blood eosinophil count greater than or equal to 200 cells per µL.

The IL-5 mediated disease may be selected from: asthma, Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) or dermatitis.

The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma.

The asthma may be severe asthma, such as severe eosinophilic asthma.

The pharmaceutical composition may comprise an aqueous liquid formulation at about pH 6.0 containing the antigen binding protein and histidine, trehalose, arginine, EDTA and/or polysorbate 80.

The pharmaceutical composition may comprise an aqueous liquid formulation at about pH 6.0 containing the antigen binding protein and about 20 mM histidine, about 180 mM trehalose, about 40 mM arginine, about 0.05 mM EDTA and about 0.02% weight of polysorbate 80 to volume.

A method of treating an IL-5 mediated disease comprising administering to a subject in need thereof an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months.

The IL-5 mediated disease may be selected from: asthma, Inflammatory bowel disease (IBD), allergic bronchopulmonary aspergillosis (ABPA), eosinophilic granulomatosis with polyangiitis (EGPA) , hypereosinophilic syndrome (HES), nasal polyposis (NP), chronic rhinosinusitis with nasal polyps (CRSwNP) or dermatitis.

The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma.

A method of treating an IL-5 mediated disease in a subject, the method comprising
a) identifying a subject with a disease selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma; and
b) administering about 100 mg to about 300 mg of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, to the subject about once every 6 months;
whereby the disease in the subject is treated.

The method may comprise administering about 100 mg of the antigen binding protein.

The IL-5 mediated disease may be eosinophilic granulomatosis with polyangiitis (EGPA) or hypereosinophilic syndrome (HES) and the method may comprise administering the antigen binding protein in an amount of about 200 mg.

The IL-5 mediated disease may be chronic rhinosinusitis with nasal polyps (CRSwNP) and the method may comprise administering the antigen binding protein in an amount of about 100 mg.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The antigen binding protein may be administered subcutaneously.

The subject may be a human subject.

The subject may have (a) an absolute blood eosinophil count at screening at start of treatment of (i) greater than or equal to 200 eosinophil cells per µL of blood or of (ii) greater than or equal to 150 eosinophil cells per µL of blood and/or (b) a blood eosinophil count which is greater than or equal to 300 eosinophils per uL of blood in the past 12 months preceding treatment.

The asthma may be severe asthma, such as severe eosinophilic asthma.

A method of decreasing an absolute blood eosinophil count in a subject, the method comprising:
a) identifying a subject having a condition selected from the group consisting of asthma, mild asthma, moderate asthma, severe asthma, mild eosinophilic asthma, moderate eosinophilic asthma, severe eosinophilic asthma, uncontrolled eosinophilic asthma, eosinophilic asthma, sub-eosinophilic asthma; and
b) administering to the subject an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region, in an amount of about 100 mg to about 300 mg, about once every 6 months, whereby the absolute blood eosinophil count in the subject is decreased.

The method may comprise administering the antigen binding protein in an amount of about 100 mg.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The antigen binding protein may be administered subcutaneously.

The subject may be a human subject.

The subject may have an absolute blood eosinophil count greater than or equal to 200 cells per µL.

The asthma may be severe asthma, such as severe eosinophilic asthma.

A pre-filled syringe comprising: a) about 100mg to about 300 mg e.g. in a total volume of about 1mL, of an antigen binding protein which binds to IL-5, the antigen binding protein comprising a heavy chain variable region having the CDRH1 amino acid sequence shown in SEQ ID NO: 5, the CDRH2 amino acid sequence shown in SEQ ID NO: 6, and the CDRH3 amino acid sequence shown in SEQ ID NO: 7; and a light chain variable region having the CDRL1 amino acid sequence shown in SEQ ID NO: 8, the CDRL2 amino acid sequence shown in SEQ ID NO: 9, and the CDRL3 amino acid sequence shown in SEQ ID NO: 10 and which also comprises a heavy chain Fc domain having a tyrosine residue at position 252, a threonine residue at position 254 and a glutamic acid residue at position 256 and wherein an amino terminus of the heavy chain Fc domain is connected to a carboxy terminus of the heavy chain variable region; and b) a pharmaceutically acceptable excipient.

The pre-filled syringe may comprise about 100 mg of the antigen binding protein.

The heavy chain variable region of the antigen binding protein may further comprise a heavy chain FR4 amino acid sequence as shown in SEQ ID NO: 19.

The heavy chain Fc domain may be an IgG1 Fc domain.

The heavy chain Fc domain may be a human IgG1 Fc domain.

The antigen binding protein may comprise: a heavy chain variable region sequence having the amino acid sequence shown in SEQ ID NO: 3; and a light chain variable region sequence having the amino acid sequence shown in SEQ ID NO: 4.

The antigen binding protein may be an antibody comprising a heavy chain having the amino acid sequence shown in SEQ ID NO: 1 and a light chain having the amino acid sequence shown in SEQ ID NO: 2.

The pre-filled syringe may be for subcutaneous administration.

The pre-filled syringe may be administered to a subject about once every 6 months.

The pre-filled syringe may be administered to a subject about once every 26 weeks (Q26W).

The pre-filled syringe may be administered to a human subject.

The human subject may have an absolute blood eosinophil count greater than or equal to 200 cells per µL.

The pre-filled syringe may comprise an aqueous liquid formulation at about pH 6.0 containing the antigen binding protein and histidine, trehalose, arginine, EDTA and/or polysorbate 80.

The pre-filled syringe may comprise an aqueous liquid formulation at about pH 6.0 containing the antigen binding protein and about 20 mM histidine, about 180 mM trehalose, about 40 mM arginine, about 0.05 mM EDTA and about 0.02% weight of polysorbate 80 to volume. The pre-filled syringe may be for use in the treatment of an IL-5 mediated disease.

The IL-5 mediated disease may be asthma.

The asthma may be selected from the group consisting of: mild asthma, moderate asthma, severe asthma, mild eosinophilic asthma, moderate eosinophilic asthma, severe eosinophilic asthma, uncontrolled eosinophilic asthma, eosinophilic asthma and sub-eosinophilic asthma.

The asthma may be severe asthma, such as severe eosinophilic asthma.

The pre-filled syringe may be provided in a safety syringe device (SSD) or an autoinjector.

### EXAMPLES

Interleukin-5 (IL-5) mediates the growth and differentiation of eosinophils in the bone marrow and their recruitment and activation within tissues (Corren, Discov. Med. 2012; 13(71): 305-312). The circulating half-life of eosinophils is around 8 to 18 hours, however, they can persist in tissues for longer (days to weeks) (Kovalszki & Weller, Eosinophilia. 2016; 43(4): 607-617). Inhibition of IL-5 will remove a key eosinophil growth factor, and given the short circulating half-life of eosinophils, it will result in a rapid reduction in the circulating population. Reduction in eosinophils has been identified as a therapeutic strategy for numerous disorders, with monoclonal antibodies (mAbs) targeting IL-5, such as mepolizumab, currently approved for the treatment of severe eosinophilic asthma and in development for other indications (Legrand & Klion, J. Allergy Clin. Immunol. Pract. 2015; 3(2): 167-174).

28Y042-7F11-1 is an extended pharmacology humanised monoclonal antibody (Immunoglobulin G1 [IgG1], kappa). It inhibits IL-5 signalling by blocking human IL-5 binding to the IL-5 receptor complex, which is expressed on the eosinophil cell surface. Treatment with anti-IL-5 monoclonal antibodies, administered every 4 weeks, is well tolerated and is approved as an add-on maintenance treatment in patients with severe eosinophilic asthma. 28Y042-7F11-1 is anticipated to confer a similar efficacy and benefit:risk profile as other IL-5-targeting monoclonal antibodies, while being administered with a longer dosing interval.

### EXAMPLE 1: Dosing study

This was a single ascending dose, first time in human (FTIH) study to investigate the safety, tolerability, immunogenicity, PK and PD of 28Y042-7F11-1, administered subcutaneously in participants with mild to moderate asthma, maintained on a low-medium daily dose of inhaled corticosteroids (ICS) or ICS/long acting β-agonist (LABA) and short acting β-agonist (SABA).

### Methods

### Study design

A summary of 28Y042-7F11-1 first-time-in-human study design is shown in Figure 1. Each participant received a single dose of 28Y042-7F11-1 or placebo, as shown in Figure 1.

### Participants

Eligible participants were required to have a blood eosinophil count of ≥200 cells/µL at screening, to facilitate investigation of reduction in blood eosinophil counts following single ascending doses of 28Y042-7F11-1 and to quantify the prolonged blood eosinophil reduction that was anticipated in humans, given the expected extended half-life and greater IL-5 affinity of 28Y042-7F11-1. A blood eosinophil pre screen assessment was performed. Participants were eligible to skip the blood eosinophil pre-screening visit and go directly to the screening visit, if they had a documented blood test result within 12 weeks before the dosing demonstrating a blood eosinophil level ≥200 cells/µL.

After completion of successful screening, 48 participants with mild to moderate asthma, with blood eosinophils ≥200 cells/µL at screening, were randomised and dosed. Participants were randomised in accordance with the randomisation schedule generated by Biostatistics, prior to the start of the study, using validated internal software. Participants in each dose cohort were allocated in a 3:1 ratio (active:placebo).

The follow-up period was up to 40 weeks after dosing, and was dose dependent, based on the predicted blood eosinophil profile. Participants in cohorts receiving 2 and 10 mg 28Y042-7F11-1 attended the clinic up to Week 32; participants in cohorts receiving 30 and 100 mg 28Y042-7F11-1 attended the clinic up to Week 36; and participants in cohort receiving 300 mg attended the clinic up to Week 40.

### Treatment

The term 'study treatment' as used herein may describe any combination of products received by the participants as per the protocol design.

**Table 6 - Identity of Investigational Product(s)**

| | **Study Treatment** | |
|---|---|---|
| **Product name:** | **28Y042-7F11-1 Injection, 150 mg/mL** | **0.9% w/v Sodium Chloride Injection (placebo)** |
| **Formulation description:** | The 28Y042-7F11-1 drug product was formulated with L-Histidine, monohydrochloride, monohydrate; L-Histidine; Trehalose dihydrate; L-Arginine hydrochloride; Disodium Edetate Ethylenediaminetetraacetic acid (EDTA); L-Methionine; and polysorbate 80 | 0.9% w/v Sodium Chloride |
| **Dosage form:** | Solution for injection | Solution for injection |
| **Unit dose strength(s)/Dosage level(s):** | 150 mg/mL (1 mL nominal volume) in a 3 mL glass vial; | Volume to match active dose |
| | Dosage levels varied per cohort. | |
| **Route of Administration** | Subcutaneous Injection. | To match active (i.e. the same volume and number of injections as the participants on 28Y042-7F11-1 in that cohort) |
| | 1 mL solution per injection. | |
| **Physical description:** | Clear or opalescent, colourless or yellow to brown liquid. Essentially free from visible particulates. | Refer to product insert |
| **Manufacturer/Source of procurement:** | GSK | Locally sourced by trial site |

### Pharmacokinetic Assessments

Blood samples for determination of 28Y042-7F11-1 plasma concentration were collected at the pharmacokinetic sampling time points designed as part of the study. Blood samples were taken via an indwelling cannula (or by direct venepuncture) and collected in a tri-potassium ethylenediaminetetraacetic acid (K3EDTA) tube. Blood sample processing (i.e., centrifugation, followed by harvesting of resultant plasma and storage at -70°C) was done within 2 hours of collection.

Concentrations of 28Y042-7F11-1 were determined in plasma samples using the currently approved bioanalytical methodology. Raw data was archived at the bioanalytical site.

### Pharmacodynamic Biomarkers

Serum samples were collected during this study to measure serum total IL-5 levels as a marker of target engagement. Additionally, blood eosinophil levels were measured as part of the haematology panel as a marker of pharmacological response. Samples were collected at time points designed as part of the study.

### Results

### Plasma 28Y042-7F11-1 Pharmacokinetic Parameters

Derived plasma 28Y042-7F11-1 PK parameters following single SC administration of different doses (2 to 300 mg) of 28Y042-7F11-1 in participants with mild to moderate asthma are summarised in Table 7.

**Table 7 - 28Y042-7F11-1 Plasma Pharmacokinetic Parameters Following Single Subcutaneous (SC) Administration of Different Doses of 28Y042-7F11-1 in Participants**

| **Parameter (units)** | **28Y042-7F11-1 SC Administration** | | | | |
|---|---|---|---|---|---|
| | **2 mg (N=6)** | **10 mg (N=6)** | **30 mg (N=9)** | **100 mg (N=9)** | **300 mg (N=6)** |
| **n** | 6 | 6 | 9 | 9 | 6 |
| **AUC(0-∞) (day*µg/mL)¹** | 24.8 (14.9, 41.3) 51.6 | 68.9 (53.9, 88.0) 23.6 | 208.3 (155.3, 279.4) 39.6 | 846.7 (800.3, 895.8) 7.3 | 1873.7 (1439.3, 2439.1) 25.5 |
| **AUC(0-t) (day*µg/mL)¹** | 18.4 (10.3, 32.7) 59.2 | 62.1 (46.4, 83.2) 28.4 | 201.4 (148.7, 272.9) 41.1 | 830.2 (784.2, 879.0) 7.4 | 1855.6 (1421.1,2423.0) 25.8 |
| **AUC(0-Week4) (day*µg/mL)¹** | 6.9 (5.5, 8.6) 22.0 | 19.7 (17.0, 22.8) 14.0 | 63.0 (46.6, 85.1) 40.6 | 292.7 (269.0, 318.5) 11.0 | 676.1 (534.3, 855.4) 22.7 |
| **AUC(0-Week12) (day*µg/mL)¹** | 16.1 (11.0, 23.5) 37.1 | 48.4 (38.2, 61.2) 22.7 | 148.8 (112.4, 197.0) 37.8 | 664.0 (624.1, 706.5) 8.1 | 1445.7 (1129.5, 1850.4) 23.8 |
| **AUC(0-Week26) (day*µg/mL)¹** | 21.8 (14.2, 33.5) 42.7 | 64.5 (50.1, 82.9) 24.3 | 199.9 (148.8, 268.5) 39.9 | 805.4 (759.1, 854.5) 7.7 | 1789.5 (1364.6, 2346.5) 26.3 |
| **%AUCex (%)¹** | 24.36 (16.91, 35.08) 35.8 | 7.77 (3.67, 16.44) 81.6 | 2.96 (1.99, 4.42) 55.6 | 1.42 (0.79, 2.54) 88.3 | 0.91 (0.64, 1.30) 35.1 |
| **Cmax (µg/mL)¹** | 0.340 (0.262, 0.441) 25.3 | 0.876 (0.724, 1.059) 18.3 | 2.810 (2.074, 3.808) 41.1 | 12.247 (10.849, 13.826) 15.9 | 28.599 (22.478, 36.387) 23.3 |
| **tmax (days)²** | 10.97 (7.0, 28.0) | 7.96 (7.0, 28.9) | 13.94 (4.0, 28.0) | 13.97 (4.0, 16.0) | 13.91 (2.0, 15.0) |
| **tlast (days)²** | 84.47 (84.0, 182.0) | 176.46 (126.0, 185.0) | 182.01 (182.0, 254.8) | 252.00 (250.0, 255.0) | 279.99 (278.0, 283.0) |
| **CL/F (L/day)¹** | 0.0807 (0.0484, 0.134) 51.6 | 0.1451 (0.114, 0.185) 23.6 | 0.1440 (0.107, 0.193) 39.6 | 0.1181 (0.112, 0.125) 7.3 | 0.1601 (0.123, 0.208) 25.5 |
| **Vz/F (L)¹** | 6.113 (4.175, 8.951) 37.6 | 9.193 (6.453, 13.097) 34.7 | 7.812 (5.795, 10.531) 40.4 | 6.630 (6.044, 7.273) 12.1 | 9.337 (6.821, 12.782) 30.6 |
| **λz (/day)¹** | 0.0132 (0.00904, 0.0193) 37.3 | 0.0158 (0.0133, 0.0187) 16.2 | 0.0184 (0.0170, 0.0200) 10.7 | 0.0178 (0.0163, 0.0195) 11.7 | 0.0171 (0.0161, 0.0183) 6.3 |
| **t½ (days)¹** | 52.53 (35.98, 76.71) 37.3 | 43.91 (37.07, 52.01) 16.2 | 37.60 (34.63, 40.82) 10.7 | 38.91 (35.59, 42.55) 11.7 | 40.42 (37.84, 43.18) 6.3 |

| | | | | | |
|---|---|---|---|---|---|
| 1. Geometric mean with 95% Confidence Interval (CI) and Variability (%CVb) presented. 2. Median (minimum-maximum) presented. AUC(0-∞): Area under the concentration-time curve from time zero (pre-dose) extrapolated to infinite time; AUC(0-t): Area under the concentration-time curve from time zero (pre-dose) to last time of quantifiable concentration within a participant across all treatments; AUC(0-Week N): Area under the concentration-time curve from time zero to Week N (where N = 4, 12 or 26); %AUCex: Percentage of AUC(0-∞) obtained by extrapolation; CL/F: Apparent clearance (following subcutaneous dosing); Cmax: Maximum observed concentration; SC: Subcutaneous administration; tlast: Time of last quantifiable concentration; tmax: Time of occurrence of Cmax; t½: Terminal phase half-life; Vz/F: Apparent volume of distribution (after subcutaneous administration); λz: Terminal phase elimination rate constant. Note: number of observations (n) = N (number of participants) in all cases. | | | | | |

Over the SC dose range 2 to 300 mg, 28Y042-7F11-1 AUC(0-∞), AUC(0-t) and Cmax increased with increasing dose of 28Y042-7F11-1. Median tmax ranged from 8 to 14 days across doses investigated. Geometric mean 28Y042-7F11-1 t½ ranged from 38 to 53 days across doses and was not dose-dependent (from 38 to 44 days, excluding the 2 mg dose for which %AUCex was >20% and the period over which the t½ was calculated was less than twice the resultant t½ in 4/6 participants). There was no evidence of target mediated disposition. There was no appreciable change in CL/F of 28Y042-7F11-1 across the 2 mg to 300 mg SC dose range investigated (with the exception of the 2 mg dose) with geometric mean values ranging from 0.0807 to 0.160 L/day (0.118 to 0.160 L/day, excluding 2 mg). Similarly, Vz/F of 28Y042-7F11-1 was consistent across doses investigated, with geometric mean values ranging from 6 to 9 L. Geometric mean extrapolated portion of AUC(0-∞) was small ranging from 0.9 to 8% (excluding the 2 mg dose [24%]).

Variability (%CVb) in 28Y042-7F11-1 systemic exposure (Cmax and AUCs) was generally low to moderate (7-59%).

### Blood Eosinophil Count

At Baseline, geometric mean blood eosinophil counts were similar between the placebo (0.359 GI/L) and dose groups (ranged from 0.288 GI/L to 0.398 GI/L), with the lowest Baseline geometric mean blood eosinophil counts measured for the 2 mg group and the highest for the 30 mg group. The blood eosinophil count reduction was evident from the first post-dose assessment (24 hours) in all dose groups (compared to little change in the placebo group) and the highest reduction was noted at Week 8 for most dose groups. Up to Week 8 there was no clear difference between dose groups in the magnitude of the blood eosinophil count reduction. However, there was a dose related difference in the duration of the reduction, with the suppression of blood eosinophils being maintained for longer with increasing dose (Figure 2).

The results of the Mixed Model Repeated Measures (MMRM) analysis of the ratio to baseline for blood eosinophil data adjusted for differences in baseline blood eosinophil counts are presented in Figure 3.

At Week 26 (6 months), blood eosinophil reduction of more than 80% from baseline was noted in the 100 mg, and 300 mg groups with adjusted geometric mean ratios to baseline blood eosinophils of 0.199 and 0.186, respectively (Table 8). The ratios of adjusted geometric means for these groups in comparison to placebo were 0.178 and 0.166, respectively (i.e., a reduction of 82% and 83%). The probability that the placebo-adjusted ratio to baseline in blood eosinophil count is less than 0.25 (i.e., greater than 75% reduction in blood eosinophil count) at the 26-week time point was 95% for the 100 mg group and 96% for the 300 mg group (Figure 3 and Table 8).

**Table 8 - Statistical Analysis of Ratio to Baseline Blood Eosinophil Data at Week 26**

| | **Placebo (N=12)** | **2 mg (N=6)** | **10 mg (N=6)** | **30 mg (N=9)** | **100 mg (N=9)** | **300 mg (N=6)** |
|---|---|---|---|---|---|---|
| n | 12 | 6 | 6 | 9 | 9 | 6 |
| LS Geometric Mean Ratio to Baseline | 1.119 | 0.776 | 0.663 | 0.317 | 0.199 | 0.186 |
| SE Logs | 0.1359 | 0.1950 | 0.1932 | 0.1596 | 0.1571 | 0.1935 |
| 95% CI | (0.855, 1.464) | (0.527, 1.141) | (0.452, 0.972) | (0.231, 0.435) | (0.146, 0.271) | (0.127, 0.273) |
| Ratio to Placebo | | 0.693 | 0.593 | 0.284 | 0.178 | 0.166 |
| SE Logs | | 0.2391 | 0.2373 | 0.2082 | 0.2071 | 0.2375 |
| 95% CI | | (0.432, 1.113) | (0.371, 0.948) | (0.188, 0.428) | (0.118, 0.268) | (0.104, 0.266) |
| Prob(Ratio to Placebo<0.16) | | <0.01 | <0.01 | <0.01 | 0.31 | 0.44 |
| Prob(Ratio to Placebo<0.25) | | <0.01 | <0.01 | 0.27 | 0.95 | 0.96 |
| Prob(Ratio to Placebo<0.5) | | 0.09 | 0.24 | >0.99 | >0.99 | >0.99 |

| | | | | | | |
|---|---|---|---|---|---|---|
| Note: Analysis was performed using an MMRM model on log-transformed data, with fixed categorical effects of treatment, planned timepoint and treatment-by-planned timepoint interaction and fixed continuous covariates of log Baseline blood eosinophil count and log Baseline blood eosinophil count-by-planned timepoint interaction. A Toeplitz covariance structure was used. Note: n is the number of participants with non-missing data at the relevant planned timepoint. | | | | | | |

Since the magnitude of reduction in blood eosinophils was similar for all groups at the early timepoints, it was not appropriate to fit the 4-parameter dose-response Emax Bayesian model at every time point. The analysis of the dose-response relationship was only conducted with the Bayesian model at the Week 26 time-point, which had measurements in all cohorts and was the primary time-point of interest. The result of this analysis is presented in Table 9.

**Table 9 - Statistical Analysis of Ratio to Baseline Blood Eosinophil Data at Week 26 (Bayesian Emax Sensitivity Analysis)**

| | **Placebo (N=12)** | **2 mg (N=6)** | **10 mg (N=6)** | **30 mg (N=9)** | **100 mg (N=9)** | **300 mg (N=6)** |
|---|---|---|---|---|---|---|
| n | 12 | 6 | 6 | 9 | 9 | 6 |
| Posterior Mean Ratio to Baseline | 1.024 | 0.886 | 0.623 | 0.338 | 0.211 | 0.171 |
| 95% CI | (0.786, 1.352) | (0.678, 1.124) | (0.453, 0.887) | (0.247, 0.451) | (0.168, 0.270) | (0.121, 0.243) |
| Ratio to Placebo | | 0.865 | 0.609 | 0.331 | 0.206 | 0.167 |
| 95% CI | | (0.663, 1.000) | (0.412, 0.993) | (0.226, 0.497) | (0.148, 0.289) | (0.107, 0.278) |
| Prob (Ratio to Placebo<0.16) | | <0.01 | <0.01 | <0.01 | 0.07 | 0.43 |
| Prob (Ratio to Placebo<0.25) | | <0.01 | <0.01 | 0.08 | 0.87 | 0.96 |
| Prob (Ratio to Placebo<0.5) | | <0.01 | 0.23 | 0.98 | >0.99 | >0.99 |

| | | | | | | |
|---|---|---|---|---|---|---|
| Note: Analysis was performed using a 4-parameter Emax Bayesian dose response model fitted on log-transformed data with log Baseline blood eosinophil count included as a covariate. Non-informative priors were used on all parameters in the model. | | | | | | |

The Bayesian analysis was supportive of the MMRM analysis, with blood eosinophil reductions from baseline of 79% and 83% being noted in the 100 mg, and 300 mg groups, respectively (Table 9). The ratios of adjusted geometric means for these groups in comparison to placebo were 0.206 and 0.167, respectively (i.e., a reduction of 79% and 83%). The probability that the placebo-adjusted ratio to baseline in blood eosinophil count is less than 0.25 (i.e., greater than 75% reduction in blood eosinophil count) at the 26-week time point was 87% for the 100 mg group and 96% for the 300 mg group (Table 9).

A second sensitivity analysis was performed after the application of windowing to ensure that assessments were taken within an acceptable timeframe of the planned visit dates and times. At Week 26, all of the treatment groups had 1 participant excluded due to time deviations. Nevertheless, results were similar to the main analysis, with a probability of 90% for the 100 mg group and 97% for the 300 mg group that the placebo-adjusted ratios to baseline in blood eosinophil count is less than 0.25 (i.e., greater than 75% reduction in blood eosinophil count) at the 26-week time point.

### Serum Total IL-5

The geometric mean serum total IL-5 (i.e., free IL-5 plus IL-5 bound to 28Y042-7F11-1) value at baseline for the placebo group (9.206 ng/L) was slightly lower than that of the dose groups (10.376 to 15.109 ng/L). In general, the majority of participants had baseline values below the limit of quantification of the assay in each dose group. From Week 1 (Day 8) onwards all participants in the dose groups had measurable concentrations of serum total IL-5. An increase in serum total IL-5 from baseline was noted for all the dose groups for all the study visits. There was however no clear dose response observed. By contrast, the geometric mean serum total IL-5 values of the placebo group remained similar to baseline during the study (Figure 4).

### Exploratory Biomarker Results

Evaluation of exploratory asthma biomarkers was performed in the study. The levels of Eosinophil Derived Neurotoxin (EDN), Eotaxin, Macrophage Derived Chemokine (MDC), Monocyte Chemotactic Protein 4 (MCP-4/CCL13) and Thymus and Activation-Regulated Chemokine (TARC/CCL17) were measured at Day -1, 29 and 127. Reduction in levels of Eosinophil Derived Neurotoxin were seen with 28Y042-7F11-1 treatment, while no changes were noted with the other markers. These results mirror findings with mepolizumab treatment using the same biomarker panel.

### Safety Results

The study assessed the safety and tolerability of single subcutaneous doses of 28Y042-7F11-1 2 mg, 10 mg, 30 mg, 100 mg, and 300 mg as compared to placebo in adult in participants with mild to moderate asthma. All the 28Y042-7F11-1 doses were well tolerated by the participants. The incidence of on-treatment adverse events was 92% on placebo and 81% in all doses. No serious adverse events or adverse events leading to withdrawal from study were reported.

### Conclusions

Over the studied dose range, 28Y042-7F11-1 was absorbed slowly with median observed tmax of 8-14 days, and distributed into an apparent volume of distribution of 6-9 L. This volume corresponds to approximate plasma and interstitial space, consistent with values reported for typical monoclonal antibodies targeting soluble ligands. After tmax, plasma concentrations of 28Y042-7F11-1 declined in a mono-exponential manner with a geometric mean t½ of 38-53 days and apparent clearance 0.0807-0.160 L/day. Compared to mepolizumab (Nucala PI), another anti IL-5 monoclonal antibody, these values represent an increase in half-life and reduction in apparent clearance of approximately two-fold. Geometric mean pharmacokinetic parameters (Vz/F, CL/F and t½) were consistent across the dose range studied and therefore independent of the dose implying 28Y042-7F11-1 PK is linear, with no evidence of target mediated disposition. Furthermore, with the exception of the 2 mg dose, there was no major deviation from dose proportionality for Cmax and AUC(0-∞) over the SC dose range 10-300 mg.

Due to the wide range of 28Y042-7F11-1 single SC doses (2 to 300 mg) investigated over an extended period of time (up to 40 weeks post-dosing) in this study, it was possible to assess the dose response of blood eosinophil counts. A marked blood eosinophil count reduction was observed from the first post-dose assessment at Day 2 (24 hours), and the highest reduction noted at Week 8 for most dose groups. While the reduction was dose independent up to Week 8, a clear dose response was evident for the return towards baseline. At Week 26 (6 months), blood eosinophil reduction compared to placebo after adjusting for baseline blood eosinophil counts was 82% and 83% in the 100 mg, and 300 mg groups, respectively. These reductions were similar to those reported with mepolizumab at the approved therapeutic dose (product information [PI]). The probability that the placebo-adjusted ratio to baseline in blood eosinophil count is <0.25 (i.e., >75% reduction in blood eosinophil count) at the 26-week time point was 95% for the 100 mg group and 96% for the 300 mg group (MMRM analysis). These probabilities exceeded the pre-defined success criterion of 80%. These results reflect the increased affinity of 28Y042-7F11-1 for IL-5 as well as the increase in half-life of the antibody.

In conclusion:
- 28Y042-7F11-1 was well tolerated in adult participants with mild/moderate asthma who received a single dose of 28Y042-7F11-1 up to 300 mg SC.
- The geometric mean terminal phase half-life (38 to 53 days) is increased approximately two-times compared with conventional IgG1 monoclonal antibodies targeting soluble ligands.
- 28Y042-7F11-1 reduced blood eosinophil counts with a clear dose response observed for the return towards baseline. At Week 26, the adjusted reduction compared to placebo was 82% and 83% in the 100 mg, and 300 mg groups, respectively. Both doses met the pre-defined success criterion for placebo-adjusted ratio to baseline blood eosinophil count at Week 26.

Using the results from this study and model-informed drug development (MIDD) principles, a dose and dosing frequency of 28Y042-7F11-1 that matches established Phase 3 mepolizumab-like blood eosinophil pharmacology most closely, with additional flexibility should a patient delay their next dose (e.g., providing 4 weeks flexibility), is 100 mg once every 6 months (or once every 26 weeks -Q26W - as the terms once every 6 months and Q26W are used interchangeably ).

### EXAMPLE 2: Phase III clinical protocols - ASTHMA

This prophetic example describes the intended Phase III clinical studies. Each placebo-controlled, exacerbation study will be 12 months in duration for investigation of two repeat dose administrations of 28Y042-7F11-1 100 mg SC and will investigate effects in adult and adolescent (≥12 years) patients with asthma and an eosinophilic phenotype on inhaled corticosteroids (ICS) plus an additional controller therapy and a history of repeat exacerbations (≥2 in the previous 12 months), a population shown to receive clinical benefit from anti-IL-5 treatment. A 12-month study allows for two repeat administrations of 28Y042-7F11-1 and is sufficiently long to assess the efficacy of 28Y042-7F11-1 100 mg SC every 26 weeks on the frequency of clinically significant exacerbations and other relevant outcomes, as well as the safety profile with adequate exposure.

Following a minimum 1 week (up to 6 week) run-in, 375 eligible subjects will be randomised 2:1 to receive 28Y042-7F11-1 100 mg SC (n=350) or matching placebo (n=175). The first injection will be administered at randomisation. Subjects will have visits at Week 2, Week 4 and then at 4 weekly intervals thereafter, with a further visit at Week 26 to administer the second injection. The last visit (End of Study visit) will occur at Week 52. Subjects will have the option to continue in a 12-month open-label extension study where subjects already receiving 28Y042-7F11-1 will receive up to 2 further additional doses and subjects previously receiving placebo will receive up to 2 doses of 28Y042-7F11-1.

Eligible subjects will be adults and adolescents (≥12 years) with a confirmed diagnosis of asthma receiving medium to high dose ICS and at least one other controller. Subjects should have uncontrolled asthma as demonstrated by ≥2 exacerbations in the previous 12 months, despite existing therapy. Subjects will be required to have a blood eosinophil level of ≥150 cells/µL at screening or ≥300 cells/µL in the 12 months prior to screening.

The primary outcome measure will be the frequency of clinically significant exacerbations (i.e. exacerbations requiring systemic corticosteroid and/or ER visit and/or hospitalisation) over the 12-month treatment period. In addition to exacerbations, other efficacy endpoints will include lung function (pre- and post-bronchodilator FEV1), asthma control (Asthma Control Questionnaire - ACQ-5), quality of life measured with the St Georges Respiratory Questionnaire (SGRQ) and diary card parameters including peak flow, rescue use, daily symptoms and nocturnal awakening due to asthma. Other measures of asthma control and response to treatment will also be assessed throughout the studies.

Blood eosinophils will be measured throughout the treatment period and sparse PK samples collected (pre-dose, Week 2, Week 12, Week 26, Week 28, Week 40 and Week 52). The study will include comprehensive safety and immunogenicity assessments to robustly characterize safety outcomes following repeat dosing. Immunogenicity will be assessed at pre-dose at Week 0, Week 2, Week 4, Week 8, Week 12, Week 26, Week 28, Week 30, Week 32, Week 36, Week 40 and Week 52.

28Y042-7F11-1 is anticipated to demonstrate a similar clinical profile to mepolizumab. This is supported by the results of the study described in Example 1 which collected pharmacokinetic, pharmacodynamic, safety and tolerability data. Single doses up to 300 mg SC (3 times the planned therapeutic dose) were evaluated in subjects with mild-moderate asthma but whose blood eosinophils were elevated (≥200 cells/µL at screening). 28Y042-7F11-1 was well tolerated with no safety signals detected. The effect on blood eosinophils was as predicted from mepolizumab accumulated pharmacology knowledge, with overall maximum blood eosinophil suppression similar to that seen with mepolizumab 100 mg SC/75 mg IV, though the period of suppression was prolonged compared with that seen previously for mepolizumab.

Therefore, in addition to the two exacerbation studies, a 12-month, supporting study enrolling approximately 1,700 adult and adolescent subjects (≥12 years) currently receiving either mepolizumab or benralizumab is also proposed. Subjects will be randomised 1:1 to either continue with their existing therapy or be switched to 28Y042-7F11-1 100 mg SC every 26 weeks. The study will aim to show that treatment with 28Y042-7F11-1 100 mg SC every 26 weeks is non-inferior to existing anti-IL-5 therapy on the rate of clinically significant exacerbations (i.e. exacerbations requiring systemic corticosteroid and/or ER visit and/or hospitalisation) and other markers of asthma control utilizing a non-inferiority design.

Following a 1 week minimum (up to 6 week maximum) run-in, 1,600 subjects will be randomised 1:1 to receive either 28Y042-7F11-1 100 mg SC once every 26 weeks (n=800) or continue with their existing therapy (n=800). At randomisation, subjects will receive two injections, either active 28Y042-7F11-1 100 mg and placebo matching mepolizumab/benralizumab (depending on the subject's prior treatment), or placebo 28Y042-7F11-1 and active mepolizumab 100 mg SC/benralizumab 30 mg SC (according to their prior treatment). Thereafter, clinic visits will be every 4 weeks, with subjects previously on mepolizumab receiving injections of mepolizumab 100 mg SC or matching placebo every 4 weeks. Subjects previously receiving benralizumab will receive injections of benralizumab 30 mg SC or matching placebo every 8 weeks. There will be a further visit at Week 26 for the administration of 28Y042-7F11-1 100 mg SC or matching placebo. The End of Treatment visit will occur at Week 52, with a follow-up visit at Week 56.

Eligible subjects will be adults and adolescents (≥12 years) currently receiving mepolizumab or benralizumab for severe asthma for at least 12 months prior to entry and have shown to have had a proven response to anti-IL-5 therapy assessed by either (1) a ≥50% reduction in exacerbation frequency, or (2) a ≥50% reduction in maintenance OCS use, or (3) no exacerbations in the past 6 months whilst receiving anti-IL-5 therapy and an ACQ-5 score of ≤1.5 at screening. Subjects must have a demonstrated clinical benefit from their current anti-IL-5 therapy either in the reduction in the frequency of exacerbations and/or the reduction in maintenance oral corticosteroid therapy prior to commencing anti-IL-5 therapy, as confirmed by the treating physician. As well as receiving anti-IL-5 therapy, subjects should be receiving moderate or high doses of inhaled corticosteroid plus another controller medication for the treatment of asthma.

The primary outcome variable will be frequency of clinically significant exacerbations over the 12-month treatment period. In addition, other efficacy measures will be lung function (pre- and post-bronchodilator FEV1), asthma control as assessed by the asthma control questionnaire (ACQ-5), quality of life using the St Georges Respiratory Questionnaire (SGRQ) and diary card parameters (symptoms, peak flow, rescue use and nocturnal awakening due to asthma). Other measures of asthma control and response to treatment will also be assessed throughout the study.

Blood eosinophils will be measured throughout the treatment period. The study will include comprehensive safety and immunogenicity assessments to robustly characterize safety outcomes following repeat dosing. Immunogenicity will be assessed pre-dose at Week 0, Week 4, Week 8, Week 12, Week 26, Week 40 and Week 52.

### Formulation

Previous studies utilized a liquid formulation of 28Y042-7F11-1 injection, 150 mg/mL, supplied as a sterile, preservative-free, single use aqueous solution in vials. The required dose levels were prepared at clinical sites through dilution with saline and then injected as a subcutaneous (SC) dose (single injection for up to 100 mg dose and 3 injections for the 300 mg dose).

Phase 3 studies will be initiated utilizing a new formulation based on the previous formulation. The changes between the previous formulation and the new formulation are that concentration of 28Y042-7F11-1 has been reduced from 150mg/mL to 100 mg/mL, methionine has been removed, and the concentration of some excipients has been reduced (Table 10).

**Table 10 - Product profile**

| **Product Profile (Design)** | |
|---|---|
| Dose presentations | 28Y042-7F11-1, 100mq/ml |
| Dose Form | Sterile liquid in prefilled syringe (PFS) assembled in safety syringe device (SSD) and autoinjector (AI) |
| Formulation | 100 mg/mL 28Y042-7F11-1 |
| | 20 mM L-histidine |
| | 180 mM Trehalose dihydrate |
| | 40 mM L-Arginine |
| | 0.05 mM Disodium edetate (EDTA) |
| | 0.02% w/v polysorbate 80 |
| | pH 6.0 |
| Deliverable Volume | 1.0 mL |
| DP Strengths | 100 mg/mL |
| Container Closure System (primary packaging) | Prefilled Syringe |
| Container Closure System (devices) | Safety Syringe Device Autoinjector |
| Route of administration | Subcutaneous injection |
| Intended delivery setting | Product will be administered by a healthcare provider in a clinical setting or at home by the patient or caregiver in a home setting |
| Drug Product Quality Criteria | Product must meet all release and stability acceptance criteria. |

| **Stability** | |
|---|---|
| Shelf life | Minimum of 24 months |
| In-use stability | Stable for minimum of 4 hours at room temperature (19°C-25°C) |
| Long Term Storage | 2-8°C, protect from light |
| Stability during transport | Transport refrigerated 2-8 ° C. |
| | Ability to withstand transportation by air, sea and ground. |

During these studies, the 28Y042-7F11-1 injection, 100 mg/mL, will be provided as a single-use, sterile liquid drug product (DP) in a clear glass pre-filled syringe (PFS) that delivers 100mg of 28Y042-7F11-1 for subcutaneous administration. The DP primary container and closure consists of a 1 mL long Type I glass siliconized barrel with a 29G thin wall x 12.7 mm stainless steel needle with a thermoplastic elastomer needle shield covered by rigid plastic shield sealed with bromobutyl elastomer rubber plugger stopper. The prefilled syringe will be assembled into a safety syringe device, BD UltraSafe Passive Plus safety device.

The 28Y042-7F11-1 formulation detailed in Table 10 has particularly good stability and the drug product was found to have remained stable following storage for up to 18 months at the recommended long-term storage temperature of 2-8°C. In addition, the drug product remained stable when stored for 1 month at -20°C, 0.5 month at 30°C/65% relative humidity (RH) and after exposure to 3 freeze-thaw cycles. A shelf life of 30 months was applied to the drug product for Injection, 100 mg/mL when stored at 2°C - 8°C (36°F - 46°F), and protected from light.

### EXAMPLE 3: Modelling to determine Dosing for Chronic Rhinosinusitis with nasal polyps (CRSwNP)

The proposed 28Y042-7F11-1 dose and dosing frequency of 100 mg every 26 weeks for the Phase 3 studies in CRSwNP was identified using model-informed drug development (MIDD) principles. 28Y042-7F11-1 dosing regimen was selected to closely match, over the entire treatment period, blood eosinophil pharmacology observed with mepolizumab in the pivotal Phase 3 trial in the CRSwNP population. No further dose-ranging of 28Y042-7F11-1 is considered necessary prior to starting the proposed Phase 3 program in CRSwNP. The simulation analysis shows that there would be limited additional benefit, in term of blood eosinophil reduction, beyond the proposed 28Y042-7F11-1 100 mg SC dose administered every 26 weeks; while lower doses would not match the desired blood eosinophils pharmacology and would thus most likely be non-efficacious.

The desired blood eosinophils pharmacology was informed by pivotal Phase 3 trials with mepolizumab. In the mepolizumab pivotal Phase 3 trial in CRSwNP, the steady-state eosinophils reduction compared to baseline was 84% following mepolizumab 100 mg SC administered every 4 weeks. This reduction translated into geometric mean (std logs) of blood eosinophils count at week 52 equal to 0.06 (0.768) GI/L in the cohort receiving mepolizumab 100 mg SC administered every 4 weeks. This level of pharmacology was associated with clinical efficacy. At the end of the 52-week treatment period, a greater proportion of participants in the mepolizumab group than the placebo group demonstrated a ≥1-point improvement in their total endoscopic NP score (50% compared with 28%, respectively), and fewer participants in the mepolizumab group than the placebo group had a worsening of their total endoscopic NP score over the same period (22% compared with 30%, respectively). For the co-primary endpoint of the change from baseline in total endoscopic NP score at Week 52, the median change in the mepolizumab group was -1.0 compared with 0 in the placebo group. There was a statistically significant improvement in this endpoint in favour of mepolizumab. Furthermore, in severe eosinophilic asthma (a disease with which CRSwNP shares many characteristics), blood eosinophils reduction with the approved mepolizumab dose of 100 mg SC administered every 4 weeks was 86% and 81%, in Phase 3 trials in which annualized exacerbation rate ratio was consistently reduced by approximately 50%. The simulation analysis shows that there would be limited additional benefit, in term of blood eosinophil reduction, beyond the proposed 28Y042-7F11-1 dosing regimen of 100 mg SC administered every 26 weeks. Furthermore, the simulation analysis shows that lower doses would not match the desired blood eosinophils pharmacology and would thus most likely be non-efficacious.

Selection of 28Y042-7F11-1 dose and dose frequency based on targeting previous mepolizumab pharmacology is both valid and expeditious since:
- 28Y042-7F11-1 targets the same IL-5 epitope as mepolizumab, it therefore follows that establishing the same reduction in blood eosinophils as mepolizumab, via the same IL-5 neutralization is expected to generate similar clinical efficacy in the CRSwNP patient population selected for these studies;
- the precedented safety profile of IL-5 neutralization is comparable to placebo.

The PK-blood eosinophils model used to support 28Y042-7F11-1 dose selection is an updated model based on the indirect exposure - eosinophil model developed on data from 16 mepolizumab studies that collected pharmacokinetic samples, blood eosinophil count measurements and covariates during mepolizumab clinical development in various eosinophilic conditions and in healthy subjects. The model was parameterised in terms of baseline blood eosinophils (KRO), rate of elimination (KOUT), maximum inhibitory effect (Imax), concentration resulting in 50% of maximum inhibitory drug effect (IC50), and Hill coefficient (GAMA). Between subject variability was included on baseline blood eosinophil and maximum drug effect using an exponential model. Measured baseline blood eosinophil count was a covariate of KRO and Imax. Disease was covariates for KRO. This model was updated, maintaining the same model structure, to reflect the longer half-life and enhanced potency of 28Y042-7F11-1 compared to mepolizumab. The model IC50 parameter, the between subject variability and the residual unexplained variability were estimated based on PK and eosinophil data from the single ascending dose Phase 1 Study with 28Y042-7F11-1 in mild to severe asthma. The impact of the baseline eosinophil levels on the maximum achievable drug inhibitory response and on model-predicted baseline blood eosinophils was assumed to be the same between mepolizumab and 28Y042-7F11-1.

Simulation of 28Y042-7F11-1 PK-blood eosinophils was based on the 28Y042-7F11-1 PK model developed on data from 28Y042-7F11-1 Phase 1 study and on the updated PK-blood eosinophils model. Simulated eosinophil levels at baseline reflected the value observed in the mepolizumab Phase 3 pivotal trial in CRSwNP (0.39 GI/L).

Model parameter values utilised for simulation were sampled from a multivariate normal distribution, centred in the parameter estimated values, with covariance reflecting the uncertainty of parameter estimates. Residual unexplained variability was included in the simulation. Results of the simulation were summarised in terms of geometric mean and standard deviation of logs and 95% prediction interval.

### EXAMPLE 4: Modelling to determine Dosing for eosinophilic granulomatosis with polyangiitis (EGPA)

The proposed 28Y042-7F11-1 dose and dosing frequency of 200 mg every 26 weeks for the Phase 3 study in EGPA was identified using model-informed drug development (MIDD) principles. 28Y042-7F11-1 dosing regimen was selected to closely match, over the entire treatment period, blood eosinophil pharmacology observed with mepolizumab in the pivotal Phase 3 trial in the EGPA population. No further dose-ranging of 28Y042-7F11-1 is considered necessary prior to starting the proposed Phase 3 program. The simulation analysis shows that there would be limited additional benefit, in term of blood eosinophil reduction, beyond the proposed 28Y042-7F11-1 200 mg SC dose administered every 26 weeks; while lower doses would not match the desired blood eosinophils pharmacology and would thus most likely be non-efficacious.

The desired blood eosinophils pharmacology was informed by the pivotal Phase 3 trial with mepolizumab in EGPA, where 32% of participants in the mepolizumab group achieved remission at both Week 36 and Week 48 (vs 3% in the placebo group). In the trial, the steady-state eosinophils reduction compared to baseline was 79% following mepolizumab 300 mg SC administered every 4 weeks. This reduction translated into geometric mean (std logs) of blood eosinophils count, at the end of the treatment period, equal to 0.038 (0.9398) GI/L in the cohort receiving mepolizumab 300 mg SC administered every 4 weeks. The simulation analysis shows that there would be limited additional benefit, in term of blood eosinophil reduction, beyond the proposed 28Y042-7F11-1 dosing regimen of 200 mg SC administered every 26 weeks. Furthermore, the simulation analysis shows that lower doses would not match the desired blood eosinophils pharmacology and would thus most likely be non-efficacious.

Selection on 28Y042-7F11-1 dose and dose frequency based on targeting previous mepolizumab pharmacology is both valid and expeditious since:
- 28Y042-7F11-1 targets the same IL-5 epitope as mepolizumab, it therefore follows that establishing the same reduction in blood eosinophils as mepolizumab, via the same IL-5 neutralization is expected to generate similar clinical efficacy in the EGPA patient population selected for this study;
- the precedented safety profile of IL-5 neutralization is comparable to placebo.

The PK-blood eosinophils model used to support 28Y042-7F11-1 dose selection is an updated model based on the indirect exposure - eosinophil model developed on data from 16 mepolizumab studies that collected pharmacokinetic samples, blood eosinophil count measurements and covariates during mepolizumab clinical development in various eosinophilic conditions and in healthy subjects. The model was parameterised in terms of baseline blood eosinophils (KRO), rate of elimination (KOUT), maximum inhibitory effect (Imax), concentration resulting in 50% of maximum inhibitory drug effect (IC50), and Hill coefficient (GAMA). Between subject variability was included on baseline blood eosinophil and maximum drug effect using an exponential model. Measured baseline blood eosinophil count was a covariate of KRO and Imax. Disease was covariates for KRO. This model was updated, maintaining the same model structure, to reflect the longer half-life and enhanced potency of 28Y042-7F11-1 compared to mepolizumab. The model IC50 parameter, the between subject variability and the residual unexplained variability were estimated based on PK and eosinophil data from the single ascending dose Phase 1 Study with 28Y042-7F11-1 in mild to severe asthma. The impact of the baseline eosinophil levels on the maximum achievable drug inhibitory response and on model-predicted baseline blood eosinophils was assumed to be the same between mepolizumab and 28Y042-7F11-1 .

Simulation of 28Y042-7F11-1 PK-blood eosinophils was based on the 28Y042-7F11-1 PK model developed on data from 28Y042-7F11-1 Phase 1 study and on the updated PK-blood eosinophils model. Simulated eosinophil levels at baseline reflected the value observed in the mepolizumab Phase 3 pivotal trial in EGPA (i.e. 0.177 GI/L) and a higher value (i.e. 1.0 GI/L), more reflective of the disease burden when oral corticosteroid are tapered, during the course of treatment, with a target oral corticosteroid dose lower than 4 mg/day.

Model parameter values utilised for simulation were sampled from a multivariate normal distribution, centred in the parameter estimated values, with covariance reflecting the uncertainty of parameter estimates. Residual unexplained variability was included in the simulation. Results of the simulation were summarised in terms of geometric mean and standard deviation of logs and 95% prediction interval.

### EXAMPLE 5: Modelling to determine Dosing for hypereosinophilic syndrome (HES)

The proposed 28Y042-7F11-1 dose and dosing frequency of 200 mg every 26 weeks for the Phase 3 study in HES was identified using model-informed drug development (MIDD) principles. 28Y042-7F11-1 dosing regimen was selected to closely match, over the entire treatment period, blood eosinophil pharmacology observed with mepolizumab in the pivotal Phase 3 trial in the HES population. No further dose-ranging of 28Y042-7F11-1 is considered necessary prior to starting the proposed Phase 3 program. The simulation analysis indeed shows that there would be limited additional benefit, in term of blood eosinophil reduction, beyond the proposed 28Y042-7F11-1 200 mg SC dose administered every 26 weeks; while lower doses would not match the desired blood eosinophils pharmacology and would thus most likely be non-efficacious.

The desired blood eosinophils pharmacology was informed by the pivotal Phase 3 trial with mepolizumab in HES, where 50% fewer participants experienced a HES flare or withdrew from the study when treated with mepolizumab compared with placebo. In the trial, the steady-state eosinophils reduction compared to baseline was 95% following mepolizumab 300 mg SC administered every 4 weeks. This reduction translated into geometric mean (std logs) of blood eosinophils count, at the end of the treatment period, equal to 0.07 (1.299) GI/L in the cohort receiving mepolizumab 300 mg SC administered every 4 weeks. The simulation analysis shows that there would be limited additional benefit, in term of blood eosinophil reduction, beyond the proposed 28Y042-7F11-1 dosing regimen of 200 mg SC administered every 26 weeks. Furthermore, the simulation analysis shows that lower doses would not match the desired blood eosinophils pharmacology and would thus most likely be non-efficacious.

Selection on 28Y042-7F11-1 dose and dosing frequency based on targeting previous mepolizumab pharmacology is both valid and expeditious since:
- 28Y042-7F11-1 targets the same IL-5 epitope as mepolizumab, it therefore follows that establishing the same reduction in blood eosinophils as mepolizumab, via the same IL-5 neutralization is expected to generate similar clinical efficacy in the HES patient population selected for this study;
- the precedented safety profile of IL-5 neutralization is comparable to placebo.

The PK-blood eosinophils model used to support 28Y042-7F11-1 dose selection is an updated model based on the indirect exposure - eosinophil model developed on data from 16 mepolizumab studies that collected pharmacokinetic samples, blood eosinophil count measurements and covariates during mepolizumab clinical development in various eosinophilic conditions and in healthy subjects. The model was parameterised in terms of baseline blood eosinophils (KRO), rate of elimination (KOUT), maximum inhibitory effect (Imax), concentration resulting in 50% of maximum inhibitory drug effect (IC50), and Hill coefficient (GAMA). Between subject variability was included on baseline blood eosinophil and maximum drug effect using an exponential model. Measured baseline blood eosinophil count was a covariate of KRO and Imax. Disease was covariates for KRO. This model was updated, maintaining the same model structure, to reflect the longer half-life and enhanced potency of 28Y042-7F11-1 compared to mepolizumab. The model IC50 parameter, the between subject variability and the residual unexplained variability were estimated based on PK and eosinophil data from the single ascending dose Phase 1 Study with 28Y042-7F11-1 in mild to severe asthma. The impact of the baseline eosinophil levels on the maximum achievable drug inhibitory response and on model-predicted baseline blood eosinophils was assumed to be the same between mepolizumab and 28Y042-7F11-1 .

Simulation of 28Y042-7F11-1 PK-blood eosinophils was based on the 28Y042-7F11-1 PK model developed on data from 28Y042-7F11-1 Phase 1 study and on the updated PK-blood eosinophils model. Simulated eosinophil levels at baseline reflected the value observed in the mepolizumab Phase 3 pivotal trial in HES (i.e. 1.46 GI/L).

Model parameter values utilised for simulation were sampled from a multivariate normal distribution, centred in the parameter estimated values, with covariance reflecting the uncertainty of parameter estimates. Residual unexplained variability was included in the simulation. Results of the simulation were summarised in terms of geometric mean and standard deviation of logs and 95% prediction interval.

### EXAMPLE 6: Phase III clinical protocols - Chronic Rhinosinusitis with nasal polyps (CRSWNP)

This prophetic example describes the intended Phase III clinical studies.

This is a randomized, double-blind, placebo-controlled, parallel group, Phase III study of depemokimab 100 mg + standard of care (SoC) in approximately 250 adults with CRSwNP. The objective of the study is to evaluate the efficacy and safety of depemokimab 100 mg, administered subcutaneously (SC) by the site staff, via a pre-filled safety syringe device every 6 months + SoC for 52 weeks. Efficacy of depemokimab will be assessed using co-primary endpoints of change from baseline in total endoscopic nasal polyp (NP) score at Week 52 and change from baseline in mean nasal obstruction VRS score from Week 49 through to Week 52.

The study population will consist of adult participants (18 years of age) with CRSwNP. In addition, participants must have an endoscopic NP score of at least 5 out of a maximum score of 8, with a minimum score of 2 in each nasal cavity. Participants must also have a prior treatment with systemic corticosteroids (SCS) anytime within the past 2 years; and/or have a medical contraindication/intolerance to SCS; and/or had a documented history of prior surgery for NP at the Screening Visit.

The study will include a 4-week run-in period followed by randomisation to a 52-week treatment period as a double-blind, placebo-controlled phase. The total study duration will be approximately 56 weeks. Randomization will be stratified based on occurrence of previous surgery for nasal polyps and country. Participants will be randomised in a 1:1 ratio into one of the two treatment groups, receiving 100 mg of depemokimab SC or placebo (both in addition to SoC) for a total of two doses (26 weeks apart).

Throughout the entire study period (run-in + treatment period), participants will be on the SoC for CRSwNP. Depending on local practice, SoC will include INCS, saline nasal douching, occasional short courses of systemic corticosteroids, and/or antibiotics. Depending on local SoC/treatment, patients treated with INCS and/ or LTRA, should if possible, continue with these treatments with no interruption nor alteration to the doses throughout the study duration. If a participant is not on INCS or LTRA prior to screening, the participant is prohibited to start any INCS or LTRA during the study.

### Key primary endpoints include:

### Change from baseline in total endoscopic NP score

Change from baseline in mean nasal obstruction VAS score during the 4 weeks prior to week 52.

Key inclusion criteria include:
Male or female 18 years of age and older inclusive, at the time of signing the informed consent.

Endoscopic bilateral NP score of at least 5 out of a maximum score of 8 (with a minimum score of 2 in each nasal cavity) assessed by the investigator.

Participants who have had at least one of the following at Visit 1:
- previous nasal surgery for the removal of NP;
- have used at least three consecutive days of systemic corticosteroids in the previous 2 years for the treatment of NP;
- medically unsuitable or intolerant to systemic corticosteroid.

Participants with severe NP symptoms defined as symptoms of nasal congestion or blockade or obstruction with moderate or severe severity (VRS score of 2 or 3) and loss of smell or rhinorrhoea (runny nose).

Presence of symptoms of chronic rhinosinusitis as described by at least 2 different symptoms for at least 12 weeks prior to Visit 1, one of which should be either nasal discharge (anterior/posterior nasal drip), plus facial pain/pressure and/or reduction or loss of smell.

### EXAMPLE 7: Phase III clinical protocols - eosinophilic granulomatosis with polyangiitis (EGPA)

This prophetic example and describes the intended Phase III clinical studies.

This is a 52-week, randomized, double-blind, double-dummy, parallel-group, multi-centre, non-inferiority study to investigate the efficacy and safety of depemokimab compared with mepolizumab in adult participants with a history of relapsing or refractory EGPA who are on stable corticosteroid therapy with or without concomitant stable immunosuppressant therapy.

Participants are required to be on a stable dose of OCS, i.e., ≥7.5 mg/day prednisolone/prednisone (but not >50 mg/day), for at least 4 weeks prior to baseline (Randomization/Visit 2). Participants receiving other immunosuppressive therapy must be on a stable dose for at least 4 weeks prior to baseline (Randomization/Visit 2) and should continue with the immunosuppressive therapy for the duration of the study.

Participants will be randomly assigned in a 1:1 ratio to receive either 200 mg depemokimab SC given as 2 x 100mg injections and administered every 26 weeks as an add on to SoC therapy or 300 mg mepolizumab SC administered every 4 weeks as an add on to standard of care (SoC therapy).

Throughout the protocol, depemokimab treatment refers to 200 mg depemokimab SC administered every 26 weeks as an add on to SoC therapy and mepolizumab treatment refers to mepolizumab SC administered every 4 weeks as an add on to SoC therapy.

The study will comprise a screening/run-in period (1 to 4 weeks), intervention period (52 weeks) and a follow-up period (4 weeks).

Approximately 192 participants will be screened to ensure 160 randomized participants (approximately 80 participants will be randomized to depemokimab 200 mg and approximately 80 participants will be randomized to mepolizumab 300 mg).

### Intervention Groups and Duration:

Participants will receive treatment with either depemokimab 200 mg every 26 weeks (at Week 0 and Week 26) or mepolizumab 300 mg every 4 weeks, administered SC using a pre-filled safety syringe (PFS) while continuing their SoC EGPA therapy. The treatment duration is 52 weeks followed by a non-treatment 4-week follow-up period. The final dose of depemokimab will be administered at Week 26 and the final dose of mepolizumab will be administered at Week 48.

### Primary endpoints:

- Remission at both Week 36 and Week 52
   - Remission defined as Birmingham Vasculitis Activity Score, BVAS = 0 and OCS dose ≤4 mg/day
EGPA Remission and relapse have 2 components:
Change in symptoms
Change in OCS dose.

Key inclusion criteria include:
- Age ≥18 years
- Diagnosed with EGPA for at least 6 months based on the history or presence of: asthma plus eosinophilia (>1.0x10⁹/L and/or >10% of leucocytes) plus at least two additional features of EGPA
- History of relapsing or refractory disease

Stable dose of oral prednisolone ≥7.5 mg/day (but not >50 mg/day) for at least 4 weeks prior to baseline. If receiving immunosuppressive (IS) therapy, must be stable from 4 weeks prior to baseline and during study.

### EXAMPLE 8: Phase III clinical protocols - hypereosinophilic syndrome (HES)

This prophetic example and describes the intended Phase III clinical studies: this is a 52-week, randomized, placebo-controlled, double-blind, parallel group, multicentre study of depemokimab in adults (≥ 18 years) with uncontrolled HES receiving SoC therapy.

The study will recruit male and female patients with a confirmed diagnosis of HES and who are on stable HES therapy for at least 4 weeks prior to randomization (Visit 2). Eligible participants must have uncontrolled HES with a history of repeated flare (≥2 flares in the previous 12 months) and blood eosinophil count of ≥1,000 cells/µL during Screening. Historical HES flares are defined as documented HES-related worsening of clinical symptoms or blood eosinophil counts requiring an escalation in therapy.

Participants will attend a Screening Visit (Visit 1) and must sign consent before initiating any Screening activity. Participants who meet the inclusion and exclusion criteria will be randomized in a 2:1 ratio to receive either depemokimab 200 mg every 6 months (given as 2 x 100mg injections) (at Week 0 and Week 26) or placebo, administered SC while continuing their SoC HES therapy.

Participant management during the study will be according to routine medical care, i.e., participants will be instructed to contact their Investigator for evaluation or seek emergency care as necessary when they experience worsening of symptoms as per their usual practice. The Investigator will use the HES Core Assessments v2 to assess for the presence of an HES flare (unscheduled Flare Visit).

Approximately 120 participants will be randomized (approximately 80 participants randomized to depemokimab 200 mg and 40 participants randomized to placebo).

Participants will receive either depemokimab 200 mg or placebo every 6 months (at Week 0 and Week 26), administered SC via a pre-filled safety syringe while continuing their SoC HES therapy. The study intervention duration is 52 weeks. The final dose of study intervention will be administered at Week 26. There will be up to 4-week additional follow-up period for all participants, i.e., 30 weeks after the last dose administered.

### Primary endpoints:

Frequency of HES flares during the 52 week treatment period.

### HES Flare definition:

HES-related clinical manifestation based on physician documented change in clinical signs/symptoms, resulting in the need for:
An increase in maintenance OCS dose
An increase or addition of any cytotoxic and/or immunosuppressive HES therapy. Receipt of 2 or more courses of blinded active OCS during treatment period (eosinophilic flare)

Key inclusion criteria include:
Age ≥18 year, Male and Female
Diagnosed of HES prior to randomization:
   blood eosinophilia of >1,500 eosinophils/µL on at least 2 occasions at ≥ 1 month interval, without a discernible non haematological secondary cause, and
   signs or symptoms of organ involvement and/or dysfunction that can be directly related to eosinophilia
   A history of 2 more or more HES flares within 12 months prior to screening:
      HES-related worsening of clinical symptoms or blood eosinophil counts requiring an addition or escalation in OCS or cytotoxic/immunosuppressive therapy.
      At least one HES flare within the past 12 months must not be related to a decrease in HES therapy during the 4 weeks prior to the flare.

### SEQUENCES

**SEQ ID NO: 1 - Full length heavy chain** 
QVTLRESGPALVKPTQTLTLTCTVSGFSLTGSSVHWVRQPPGKGLEWLGVIWASGGTDYNSALMS
RLSISKDTSRNQVVLTMTNMDPVDTATYYCARDPPSGLLRLDYWGRGTLVTVSSASTKGPSVFPLAPSSKST
SGGTAALGCLVKDYFPEPVTVSWNSGALTSGVHTFPAVLQSSGLYSLSSVVTVPSSSLGTQTYICNVNHKPSN
TKVDKRVEPKSCDKTHTCPPCPAPELLGGPSVFLFPPKPKDTLYITREPEVTCVVVDVSHEDPEVKFNWYVDG
VEVHNAKTKPREEQYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDKSRWQQGNVFSC
SVMHEALHNHYTQKSLSLSPGK
**SEQ ID NO: 2** - **Full length light chain** 
DIVMTQSPDSLAVSLGERATINCKSSQSLLNSGNQKNYLAWYQQKPGQPPKLLIYGASTRESGVPD
RFSGSGSGTDFTLTISSLQAEDVAVYYCQNVHSFPFTFGGGTKLEIKRTVAAPSVFIFPPSDEQLKSGTASVVC
 
LLNNFYPREAKVQWKVDNALQSGNSQESVTEQDSKDSTYSLSSTLTLSKADYEKHKVYACEVTHQGLSSPVT
KSFNRGEC
**SEQ ID NO: 3 - VH sequence** 
QVTLRESGPALVKPTQTLTLTCTVSGFSLTGSSVHWVRQPPGKGLEWLGVIWASGGTDYNSALMS
RLSISKDTSRNQVVLTMTNMDPVDTATYYCARDPPSGLLRLDYWGRGTLVTVSS
**SEQ ID NO: 4 - VL sequence** 
DIVMTQSPDSLAVSLGERATINCKSSQSLLNSGNQKNYLAWYQQKPGQPPKLLIYGASTRESGVPD
RFSGSGSGTDFTLTISSLQAEDVAVYYCQNVHSFPFTFGGGTKLEIKR
**SEQ ID NO: 5** - **CDRH1**
   GSSVH
**SEQ ID NO: 6** - **CDRH2**
   VIWASGGTDYNSALMS
**SEQ ID NO: 7** - **CDRH3**
   DPPSGLLRLDY
**SEQ ID NO: 8** - **CDRL1**
   KSSQSLLNSGNQKNYLA
**SEQ ID NO: 9** - **CDRL2**
   GASTRES
**SEQ ID NO: 10** - **CDRL3**
   QNVHSFPFT
**SEQ ID NO: 11 - Human IL-5 (mature protein)** 
IPTEIPTSALVKETLALLSTHRTLLIANETLRIPVPVHKNHQLCTEEIFQGIGTLESQTVQGGTVERLF
KNLSLIKKYIDGQKKKCGEERRRVNQFLDYLQEFLGVMNTEWIIES
**SEQ ID NO: 12 - Human IL-5 Receptor Subunit Alpha Isoform 1 (mature protein)** 
DLLPDEKISLLPPVNFTIKVTGLAQVLLQWKPNPDQEQRNVNLEYQVKINAPKEDDYETRITESKCV
TILHKGFSASVRTILQNDHSLLASSWASAELHAPPGSPGTSIVNLTCTTNTTEDNYSRLRSYQVSLHCTWLVG
TDAPEDTQYFLYYRYGSWTEECQEYSKDTLGRNIACWFPRTFILSKGRDWLAVLVNGSSKHSAIRPFDQLFA
LHAIDQINPPLNVTAEIEGTRLSIQWEKPVSAFPIHCFDYEVKIHNTRNGYLQIEKLMTNAFISIIDDLSKYDVQ
VRAAVSSMCREAGLWSEWSQPIYVGNDEHKPLREWFVIVIMATICFILLILSLICKICHLWIKLFPPIPAPKSNI
KDLFVTTNYEKAGSSETEIEVICYIEKPGVETLEDSVF
**SEQ ID NO: 13 - DNA encoding full length heavy chain with leader sequence** 
atgggctggtcctgcatcatcctgtttctggtggccaccgccaccggtgtgcacagccaggtgaccctgagggagagcggccc
cgccctggtgaagcccacacagaccctcactctgacctgcaccgtgagcggcttcagcctgaccggctctagcgtccactgggtgaggca
gccccccggcaagggcctggagtggctgggcgtgatctgggcaagcggggggacggactacaactcggccctgatgagcaggctctcc
atcagcaaggacaccagccggaaccaggtggtgctgaccatgaccaacatggaccccgtggacaccgccacctattactgcgccaggg
accctccctccggcctgctgaggctggactactggggcaggggaacactagtgaccgtgtccagcgccagcaccaagggccccagcgtg
ttccccctggcccccagcagcaagagcaccagcggcggcacagccgccctgggctgcctggtgaaggactacttccccgagcccgtgac
cgtgtcctggaacagcggagccctgaccagcggcgtgcacaccttccccgccgtgctgcagagcagcggcctgtacagcctgagcagcg
tggtgaccgtgcccagcagcagcctgggcacccagacctacatctgtaacgtgaaccacaagcccagcaacaccaaggtggacaagcg
ggtggagcccaagagctgtgacaagacccacacctgccccccctgccctgcccccgagctgctgggaggccccagcgtgttcctgttccc
ccccaagcctaaggacaccctgtacatcaccagagaacccgaggtgacctgtgtggtggtggatgtgagccacgaggaccctgaggtga
agttcaactggtacgtggacggcgtggaggtgcacaatgccaagaccaagcccagggaggagcagtacaacagcacctaccgggtgg
tgtccgtgctgaccgtgctgcaccaggattggctgaacggcaaggagtacaagtgtaaggtgtccaacaaggccctgcctgcccctatcg
agaaaaccatcagcaaggccaagggccagcccagagagccccaggtgtacaccctgccccctagcagagaggagatgaccaagaac
caggtgtccctgacctgcctggtgaagggcttctaccccagcgacatcgccgtggagtgggagagcaacggccagcccgagaacaacta
caagaccaccccccctgtgctggacagcgatggcagcttcttcctgtacagcaagctgaccgtggacaagagcagatggcagcagggca
acgtgttcagctgctccgtgatgcacgaggccctgcacaatcactacacccagaagagcctgagcctgtcccctggcaag
**SEQ ID NO: 14** - **DNA encoding full length light chain with leader sequence** 
atgggctggtcctgcatcatcctgtttctggtggccaccgccaccggtgtgcacagcgacatcgtgatgacccagtctcccgatt
cactggccgtgagcctgggcgagagggccaccatcaactgcaagagcagccagagcctcctgaacagcggcaaccagaagaactacc
tggcctggtaccagcagaaacccggccagccccccaagctgctgatctatggcgcctccaccagggagagcggcgtgccagacaggttt
agcggcagcggcagcggcaccgacttcaccctgacaatcagcagcctgcaggccgaggacgtggccgtgtactactgccagaacgtcc
acagcttccccttcaccttcggcgggggaaccaagctggagatcaagcgtacggtggccgcccccagcgtgttcatcttcccccccagcga
tgagcagctgaagagcggcaccgccagcgtggtgtgtctgctgaacaacttctacccccgggaggccaaggtgcagtggaaggtggac
aatgccctgcagagcggcaacagccaggagagcgtgaccgagcaggacagcaaggactccacctacagcctgagcagcaccctgacc
 
ctgagcaaggccgactacgagaagcacaaggtgtacgcctgtgaggtgacccaccagggcctgtccagccccgtgaccaagagcttca
accggggcgagtgc
**SEQ ID NO: 15** - **DNA encoding heavy chain variable region** 
caggtgaccctgagggagagcggccccgccctggtgaagcccacacagaccctcactctgacctgcaccgtgagcggcttca
gcctgaccggctctagcgtccactgggtgaggcagccccccggcaagggcctggagtggctgggcgtgatctgggcaagcgggggga
cggactacaactcggccctgatgagcaggctctccatcagcaaggacaccagccggaaccaggtggtgctgaccatgaccaacatgga
ccccgtggacaccgccacctattactgcgccagggaccctccctccggcctgctgaggctggactactggggcaggggaacactagtgac
cgtgtccagc
**SEQ ID NO: 16** - **DNA encoding light chain variable region** 
gacatcgtgatgacccagtctcccgattcactggccgtgagcctgggcgagagggccaccatcaactgcaagagcagccag
agcctcctgaacagcggcaaccagaagaactacctggcctggtaccagcagaaacccggccagccccccaagctgctgatctatggcgc
ctccaccagggagagcggcgtgccagacaggtttagcggcagcggcagcggcaccgacttcaccctgacaatcagcagcctgcaggcc
gaggacgtggccgtgtactactgccagaacgtccacagcttccccttcaccttcggcgggggaaccaagctggagatcaagcgt
**SEQ ID NO: 17** - **DNA encoding full length heavy chain** 
caggtgaccctgagggagagcggccccgccctggtgaagcccacacagaccctcactctgacctgcaccgtgagcggcttca
gcctgaccggctctagcgtccactgggtgaggcagccccccggcaagggcctggagtggctgggcgtgatctgggcaagcgggggga
cggactacaactcggccctgatgagcaggctctccatcagcaaggacaccagccggaaccaggtggtgctgaccatgaccaacatgga
ccccgtggacaccgccacctattactgcgccagggaccctccctccggcctgctgaggctggactactggggcaggggaacactagtgac
cgtgtccagcgccagcaccaagggccccagcgtgttccccctggcccccagcagcaagagcaccagcggcggcacagccgccctgggc
tgcctggtgaaggactacttccccgagcccgtgaccgtgtcctggaacagcggagccctgaccagcggcgtgcacaccttccccgccgtg
ctgcagagcagcggcctgtacagcctgagcagcgtggtgaccgtgcccagcagcagcctgggcacccagacctacatctgtaacgtgaa
ccacaagcccagcaacaccaaggtggacaagcgggtggagcccaagagctgtgacaagacccacacctgccccccctgccctgccccc
gagctgctgggaggccccagcgtgttcctgttcccccccaagcctaaggacaccctgtacatcaccagagaacccgaggtgacctgtgtg
gtggtggatgtgagccacgaggaccctgaggtgaagttcaactggtacgtggacggcgtggaggtgcacaatgccaagaccaagccca
gggaggagcagtacaacagcacctaccgggtggtgtccgtgctgaccgtgctgcaccaggattggctgaacggcaaggagtacaagtg
taaggtgtccaacaaggccctgcctgcccctatcgagaaaaccatcagcaaggccaagggccagcccagagagccccaggtgtacacc
ctgccccctagcagagaggagatgaccaagaaccaggtgtccctgacctgcctggtgaagggcttctaccccagcgacatcgccgtgga
gtgggagagcaacggccagcccgagaacaactacaagaccaccccccctgtgctggacagcgatggcagcttcttcctgtacagcaagc
tgaccgtggacaagagcagatggcagcagggcaacgtgttcagctgctccgtgatgcacgaggccctgcacaatcactacacccagaa
gagcctgagcctgtcccctggcaag
**SEQ ID NO: 18** - **DNA encoding full length light chain** 
gacatcgtgatgacccagtctcccgattcactggccgtgagcctgggcgagagggccaccatcaactgcaagagcagccag
agcctcctgaacagcggcaaccagaagaactacctggcctggtaccagcagaaacccggccagccccccaagctgctgatctatggcgc
ctccaccagggagagcggcgtgccagacaggtttagcggcagcggcagcggcaccgacttcaccctgacaatcagcagcctgcaggcc
gaggacgtggccgtgtactactgccagaacgtccacagcttccccttcaccttcggcgggggaaccaagctggagatcaagcgtacggt
ggccgcccccagcgtgttcatcttcccccccagcgatgagcagctgaagagcggcaccgccagcgtggtgtgtctgctgaacaacttctac
ccccgggaggccaaggtgcagtggaaggtggacaatgccctgcagagcggcaacagccaggagagcgtgaccgagcaggacagca
aggactccacctacagcctgagcagcaccctgaccctgagcaaggccgactacgagaagcacaaggtgtacgcctgtgaggtgaccca
ccagggcctgtccagccccgtgaccaagagcttcaaccggggcgagtgc
**SEQ ID NO: 19** - **Heavy chain FR4 sequence**
   WGRGTLVTVSS

## Claims

1. An antibody which binds to IL-5 comprising a heavy chain and a light chain, wherein the heavy chain comprises a heavy chain variable region sequence having the amino acid sequence shown in SEQ ID NO: 3; and the light chain comprises a light chain variable region sequence having the amino acid sequence shown in SEQ ID NO: 4, wherein the antibody comprises a heavy chain Fc domain having a tyrosine residue at position 252, a threonine residue at position 254 and a glutamic acid residue at position 256, wherein the heavy chain Fc domain is a human IgG1 Fc domain, for use in the treatment of an IL-5 mediated disease, wherein the antibody is to be administered subcutaneously to a human subject at a dose of about 100 mg to about 300 mg, at a frequency of about once every 6 months or once every 26 weeks (Q26W).

2. The antibody for use according to claim 1, wherein the antibody is to be administered at a dose of about 100 mg.

3. The antibody for use according to claim 1 or 2, wherein the antibody comprises a heavy chain having the amino acid sequence shown in SEQ ID NO: 1 and a light chain having the amino acid sequence shown in SEQ ID NO: 2.

4. The antibody for use according to any one of claims 1 to 3, wherein the human subject has (a) an absolute blood eosinophil count at screening at start of treatment of (i) greater than or equal to 200 eosinophil cells per µL of blood or of (ii) greater than or equal to 150 eosinophil cells per µL of blood and/or (b) a blood eosinophil count which is greater than or equal to 300 eosinophils per uL of blood in the past 12 months preceding treatment.

5. The antibody for use according to any one of claims 1 to 4, wherein the antibody is in a pharmaceutical composition.

6. The antibody for use according to claim 5, wherein the pharmaceutical composition comprises an aqueous liquid formulation at about pH 6.0 containing the antibody and histidine, trehalose, arginine, EDTA and/or polysorbate 80.

7. The antibody for use according to any one of claims 1 to 6, wherein the IL-5 mediated disease is asthma.

8. The antibody for use according to claim 7, wherein the asthma is selected from the group consisting of: mild asthma, moderate asthma, severe asthma, eosinophilic asthma, mild eosinophilic asthma, moderate eosinophilic asthma, severe eosinophilic asthma, uncontrolled eosinophilic asthma, eosinophilic asthma and sub-eosinophilic asthma.

9. The antibody for use according to claim 8, wherein the asthma is severe asthma.

10. The antibody for use according to claim 9, wherein the asthma is severe eosinophilic asthma.

11. The antibody for use according to any one of claims 7 to 10, wherein the antibody is to be administered at a dose of 100 mg once every 6 months or once every 26 weeks (Q26W).

12. The antibody for use according to any one of claims 1 to 11, wherein the antibody is to be administered in a pre-filled syringe.

13. The antibody for use according to claim 12, wherein the pre-filled syringe is provided in a safety syringe device (SSD) or an autoinjector.

14. The antibody for use according to any one of claims 1 to 13, wherein the human subject is an adult or adolescent (≥12 years).

## Patentansprüche

1. Antikörper, der an IL-5 bindet, umfassend eine schwere Kette und eine leichte Kette, wobei die schwere Kette eine Sequenz der variablen Region der schweren Kette umfasst, die die in SEQ ID NO: 3 gezeigte Aminosäuresequenz hat; und die leichte Kette eine Sequenz der variablen Region der leichten Kette umfasst, die die in SEQ ID NO: 4 gezeigte Aminosäuresequenz hat, wobei der Antikörper eine Fc-Domäne der schweren Kette umfasst, die einen Tyrosinrest an Position 252, einen Threoninrest an Position 254 und einen Glutaminsäurerest an Position 256 hat, wobei die Fc-Domäne der schweren Kette eine humane IgG1-Fc-Domäne ist, zur Verwendung bei der Behandlung einer IL-5-vermittelten Erkrankung ("IL5-mediated disease"), wobei der Antikörper einem menschlichen Subjekt subkutan in einer Dosis von etwa 100 mg bis etwa 300 mg in einer Frequenz von etwa einmal alle 6 Monate oder einmal alle 26 Wochen (Q26W) zu verabreichen ist.

2. Antikörper zur Verwendung gemäß Anspruch 1, wobei der Antikörper in einer Dosis von etwa 100 mg zu verabreichen ist.

3. Antikörper zur Verwendung gemäß Anspruch 1 oder 2, wobei der Antikörper eine schwere Kette mit der in SEQ ID NO: 1 gezeigten Aminosäuresequenz und eine leichte Kette mit der in SEQ ID NO: 2 gezeigten Aminosäuresequenz umfasst.

4. Antikörper zur Verwendung gemäß irgendeinem der Ansprüche 1 bis 3, wobei das menschliche Subjekt (a) eine absolute Blut-Eosinophilenzahl ("*blood eosinophil count"*) bei der Untersuchung zu Beginn der Behandlung von (i) größer oder gleich 200 eosinophile Zellen pro µL Blut oder von (ii) größer als oder gleich 150 eosinophile Zellen pro µL Blut und/oder (b) eine Blut-Eosinophilenzahl die größer als oder gleich 300 Eosinophilen pro µL Blut ist, in den letzten 12 Monaten vor der Behandlung aufweist.

5. Antikörper zur Verwendung gemäß irgendeinem der Ansprüche 1 bis 4, wobei der Antikörper in einer pharmazeutischen Zusammensetzung ist.

6. Antikörper zur Verwendung gemäß Anspruch 5, wobei die pharmazeutische Zusammensetzung eine wässrige Flüssigkeitsformulierung mit einem pH-Wert von etwa 6,0 umfasst, die den Antikörper und Histidin, Trehalose, Arginin, EDTA und/oder Polysorbat 80 enthält.

7. Antikörper zur Verwendung gemäß irgendeinem der Ansprüche 1 bis 6, wobei die IL-5-vermittelte Erkrankung Asthma ist.

8. Antikörper zur Verwendung gemäß Anspruch 7, wobei das Asthma aus der Gruppe ausgewählt ist, bestehend aus: leichtem Asthma, mittelschwerem Asthma, schwerem Asthma, eosinophilem Asthma, leichtem eosinophilem Asthma, mittelschwerem eosinophilem Asthma, schwerem eosinophilem Asthma, unkontrolliertem eosinophilem Asthma, eosinophilem Asthma und sub-eosinophilem Asthma.

9. Antikörper zur Verwendung gemäß Anspruch 8, wobei das Asthma schweres Asthma ist.

10. Antikörper zur Verwendung gemäß Anspruch 9, wobei das Asthma schweres eosinophiles Asthma ist.

11. Antikörper zur Verwendung gemäß irgendeinem der Ansprüche 7 bis 10, wobei der Antikörper in einer Dosis von 100 mg einmal alle 6 Monate oder einmal alle 26 Wochen (Q26W) zu verabreichen ist.

12. Antikörper zur Verwendung gemäß irgendeinem der Ansprüche 1 bis 11, wobei der Antikörper in einer vorgefüllten Spritze zu verabreichen ist.

13. Antikörper zur Verwendung gemäß Anspruch 12, wobei die vorgefüllte Spritze in einer Sicherheitsspritzenvorrichtung ("safety syringe device", SSD) oder einem Autoinjektor bereitgestellt wird.

14. Antikörper zur Verwendung gemäß irgendeinem der Ansprüche 1 bis 13, wobei das menschliche Subjekt ein Erwachsener oder Jugendlicher (≥ 12 Jahre) ist.

## Revendications

1. Anticorps qui se lie à l'IL-5 comprenant une chaîne lourde et une chaîne légère, dans lequel la chaîne lourde comprend une séquence de région variable de chaîne lourde ayant la séquence d'acide aminé représentée dans SEQ ID NO: 3 ; et la chaîne légère comprend une séquence de région variable de chaîne légère ayant la séquence d'acide aminé représentée dans SEQ ID NO: 4, dans lequel l'anticorps comprend un domaine Fc de chaîne lourde ayant un résidu de tyrosine à la position 252, un résidu de thréonine à la position 254 et un résidu d'acide glutamique à la position 256, dans lequel le domaine Fc de chaîne lourde est un domaine Fc d'IgG1 humaine, destiné à être utilisé dans le traitement d'une maladie médiée par l'IL-5, dans lequel l'anticorps doit être administré par voie sous-cutanée à un sujet humain à une dose d'environ 100 mg à environ 300 mg, à une fréquence d'environ une fois tous les 6 mois ou une fois toutes les 26 semaines (Q26W).

2. Anticorps destiné à être utilisé selon la revendication 1, dans lequel l'anticorps doit être administré à une dose d'environ 100 mg.

3. Anticorps destiné à être utilisé selon la revendication 1 ou la revendication 2, dans lequel l'anticorps comprend une chaîne lourde ayant la séquence d'acide aminé représentée dans SEQ ID NO: 1 et une chaîne légère ayant la séquence d'acide aminé représentée dans SEQ ID NO: 2.

4. Anticorps destiné à être utilisé selon l'une quelconque des revendications 1 à 3, dans lequel le sujet humain a (a) une numération sanguine absolue d'éosinophiles lors d'un criblage au début d'un traitement (i) supérieure ou égale à 200 cellules éosinophiles par µL de sang ou (ii) supérieure ou égale à 150 cellules éosinophiles par µL de sang et/ou (b) une numération sanguine d'éosinophiles qui est supérieure ou égale à 300 éosinophiles par µL de sang dans les 12 mois passés précédant le traitement.

5. Anticorps destiné à être utilisé selon l'une quelconque des revendications 1 à 4, dans lequel l'anticorps est une composition pharmaceutique.

6. Anticorps destiné à être utilisé selon la revendication 5, dans lequel la composition pharmaceutique comprend une formulation liquide aqueuse à un pH d'environ 6,0 contenant l'anticorps et de l'histidine, du tréhalose, de l'arginine, de l'EDTA et/ou du polysorbate 80.

7. Anticorps destiné à être utilisé selon l'une quelconque des revendications 1 à 6, dans lequel la maladie médiée par l'IL-5 est l'asthme.

8. Anticorps destiné à être utilisé selon la revendication 7, dans lequel l'asthme est sélectionné dans le groupe constitué de : l'asthme léger, l'asthme modéré, l'asthme sévère, l'asthme éosinophile, l'asthme éosinophile léger, l'asthme éosinophile modéré, l'asthme éosinophile sévère, l'asthme éosinophile non contrôlé, l'asthme éosinophile et l'asthme sous-éosinophile.

9. Anticorps destiné à être utilisé selon la revendication 8, dans lequel l'asthme est l'asthme sévère.

10. Anticorps destiné à être utilisé selon la revendication 9, dans lequel l'asthme est l'asthme éosinophile sévère.

11. Anticorps destiné à être utilisé selon l'une quelconque des revendications 7 à 10, dans lequel l'anticorps doit être administré à une dose de 100 mg une fois tous les 6 mois ou une fois toutes les 26 semaines (Q26W).

12. Anticorps destiné à être utilisé selon l'une quelconque des revendications 1 à 11, dans lequel l'anticorps doit être administré dans une seringue préremplie.

13. Anticorps destiné à être utilisé selon la revendication 12, dans lequel la seringue préremplie est disposée dans un dispositif de seringue de sécurité (SSD) ou dans un auto-injecteur.

14. Anticorps destiné à être utilisé selon l'une quelconque des revendications 1 à 13, dans lequel le sujet humain est un adulte ou un adolescent (≥ 12 ans).
